# FASCICULE DE BREVET EUROPEEN

(11) **EP 3 368 679 B1**
(45) Date de publication et mention de la délivrance du brevet: **04.12.2019**
(21) Numéro de dépôt: 16809939.8
(22) Date de dépôt: 27.10.2016
(51) Int. Cl.: C12P 7/22, C12R 1/645

(54) **PROCÉDÉ DE PRÉPARATION D'UN COMPOSÉ VINYLPHÉNOLIQUE À PARTIR D'UN ACIDE HYDROXYCINNAMIQUE PRÉCURSEUR ISSU D'UN TOURTEAU D'OLÉAGINEUX**
VERFAHREN ZUR HERSTELLUNG EINER VINYLPHENOLVERBINDUNG AUS EINER VON EINEM ÖLSAATKUCHEN ABGELEITETEN VORLÄUFER-HYDROXYZIMTSÄURE
PROCESS FOR PREPARING A VINYLPHENOLIC COMPOUND FROM A PRECURSOR HYDROXYCINNAMIC ACID DERIVED FROM AN OILSEED CAKE

(30) Priorité: 29.10.2015 FR 1560393
(43) Date de publication de la demande: 05.09.2018
(73) Titulaire: Institut National de la Recherche Agronomique, 75007 Paris Cedex 07 (FR); Terres Inovia, 75008 Paris 8 (FR); Terres Univia l'Interprofession des Huiles et Protéines Végétales, 75008 Paris 8 (FR); Université d'Aix Marseille, 13007 Marseille 7 (FR)
(72) Inventeur: LOMASCOLO, Anne, 13008 Marseille (FR); FINE, Frédéric, 33160 St Aubin De Medoc (FR); PEYRONNET, Corinne, 78430 Louveciennes (FR); SIGOILLOT, Jean-Claude, 83140 Six Fours Les Plages (FR); ODINOT, Elise, 13012 Marseille (FR); NAVARRO, David, 13009 Marseille (FR)
(74) Mandataire: Ipside
(86) Numéro de dépôt international: PCT/FR2016/052792
(87) Numéro de publication internationale: WO 2017/072450

(56) Documents cités:
- FR-A1- 2 792 334
- KRISTA L. MORLEY ET AL: "Antioxidant canolol production from a renewable feedstock via an engineered decarboxylase", GREEN CHEMISTRY, vol. 15, no. 12, 1 janvier 2013 (2013-01-01), page 3312, XP055281729, GB ISSN: 1463-9262, DOI: 10.1039/c3gc40748a cité dans la demande
- HONGFEI HU ET AL: "An organic solvent-tolerant phenolic acid decarboxylase from Bacillus licheniformis for the efficient bioconversion of hydroxycinnamic acids to vinyl phenol derivatives", APPLIED MICROBIOLOGY AND BIOTECHNOLOGY, vol. 99, no. 12, 31 décembre 2014 (2014-12-31), pages 5071-5081, XP055281502, DE ISSN: 0175-7598, DOI: 10.1007/s00253-014-6313-3 cité dans la demande
- JOS MANUEL SALGADO ET AL: "Purification of ferulic acid solubilized from agroindustrial wastes and further conversion into 4-vinyl guaiacol byusing solid state fermentation", INDUSTRIAL CROPS AND PRODUCTS, ELSEVIER, NL, vol. 39, 14 février 2012 (2012-02-14), pages 52-61, XP028478409, ISSN: 0926-6690, DOI: 10.1016/J.INDCROP.2012.02.014 [extrait le 2012-02-22]
- SHO-ICHI TSUJIYAMA ET AL: "Formation of 4-Vinyl Guaiacol as an Intermediate in Bioconversion of Ferulic Acid by Schizophyllum commune", BIOSCIENCE BIOTECHNOLOGY BIOCHEMISTRY., vol. 72, no. 1, 23 janvier 2008 (2008-01-23), pages 212-215, XP055281742, TOKYO, JAPAN ISSN: 0916-8451, DOI: 10.1271/bbb.60606
- L V MABINYA ET AL: "Bioconversion of ferulic acid and 4-vinylguaiacol by a white-rot fungus isolated from decaying wood", AFRICAN JOURNAL OF BIOTECHNOLOGY, vol. 9, no. 13, 1 janvier 2010 (2010-01-01), pages 1955-1958, XP055281649, DOI: 10.5897/AJB09.959
- JOHN HOWARD BIRKINSHAW ET AL: "5. BIOCHEMISTRY OF THE WOOD-ROTTING FUNGI I. METABOLIC PRODUCTS OF LENTINUS LEPIDEUS FR", BIOCHEMICAL JOURNAL., vol. 34, no. 1, 1 janvier 1940 (1940-01-01), pages 82-88, XP055281658,
- SHASHANK MISHRA ET AL: "Transformation of ferulic acid to 4-vinyl guaiacol as a major metabolite: a microbial approach", REVIEWS IN ENVIRONMENTAL SCIENCE AND BIO-TECHNOLOGY, vol. 13, no. 4, 12 décembre 2014 (2014-12-12), pages 377-385, XP055339836, NL ISSN: 1569-1705, DOI: 10.1007/s11157-014-9348-0

### SEQUENCE LISTING

<110> INSTITUT NATIONAL DE LA RECHERCHE AGRONOMIQUE
   TERRES INOVIA
   TERRES UNIVIA L'INTERPROFESSION DES HUILES ET PROTEINES
   VEGETALES
   UNIVERSITE D'AIX MARSEILLE
<120> PROCÉDÉ DE PRÉPARATION D'UN COMPOSÉ VINYLPHÉNOLIQUE À PARTIR D'UN ACIDE HYDROXYCINNAMIQUE PRÉCURSEUR ISSU D'UN TOURTEAU D'OLÉAGINEUX
<130> 31482 WO
<150> FR 1560393
   <151> 2015-10-29
<160> 6
<170> Patent In version 3.5
<210> 1
   <211> 162
   <212> PRT
   <213> Neolentinus lepideus
<400> 1
   Met Ser His Glu Gly Ala Thr Ser Glu Glu Phe Lys Gln Ile Glu Gly
   1               5                   10                  15
   Lys Arg Phe Lys Tyr Thr Tyr Gly Leu Gly Trp Thr Tyr Glu Met Tyr
               20                  25                  30
   Phe Arg Ser Leu Thr Arg Cys Val Tyr Arg Ile Leu Ser Gly Pro Leu
           35                  40                  45
   Ala Gly Arg Val Asn Phe Gln His Ala His Tyr Gln Lys Ile Arg Asp
       50                  55                  60
   Asn Val Trp Gln Cys Ser Trp Leu Glu Glu Thr Gly Thr Val Val Ser
   65                  70                  75                  80
   Met Val Val Asp Phe Asp Gln Gln Thr Val Lys Thr Phe Ala Thr Phe
                   85                  90                  95
   Ser Arg Gly His Trp Asp Leu Pro Asp Gln Ala Lys Gly Trp Lys Arg
               100                 105                 110
   Asn Pro Glu Asp Met Ala Arg Trp Arg Thr Leu Ala Gln Lys Gly Lys
           115                 120                 125
   Asp Gln Ala Asp Lys His Val Leu Val Glu His Ala Lys Met Ser Glu
       130                 135                 140
   Leu Thr Ser Gly Gln Gly Asp Leu Pro Asp Ile Asp Asp Ser Trp Glu
   145                 150                 155                 160
   Thr Met
<210> 2
   <211> 17
   <212> PRT
   <213> Neolentinus lepideus
<400> 2
   Ser His Glu Gly Ala Thr Ser Glu Glu Phe Lys Gln Ile Glu Gly Lys
   1               5                   10                  15
   Arg
<210> 3
   <211> 9
   <212> PRT
   <213> Neolentinus lepideus
<220>
   <221> misc_feature
   <222> (2)..(2)
   <223> Xaa can be any naturally occurring amino acid
<220>
   <221> misc_feature
   <222> (6)..(6)
   <223> Xaa can be any naturally occurring amino acid
<400> 3
   Ile Xaa Ser Gly Pro Xaa Ala Gly Arg
   1               5
<210> 4
   <211> 31
   <212> PRT
   <213> Neolentinus lepideus
<220>
   <221> misc_feature
   <222> (26)..(26)
   <223> Xaa can be any naturally occurring amino acid
<400> 4
   Val Val Ser Met Val Val Asp Phe Asp Gln Gln Thr Val Lys Thr Phe
   1               5                   10                  15
   Ala Thr Phe Ser Arg Gly His Trp Asp Xaa Pro Asp Gln Ala Lys
               20                  25                  30
<210> 5
   <211> 15
   <212> PRT
   <213> Neolentinus lepideus
<220>
   <221> misc_feature
   <222> (10)..(10)
   <223> Xaa can be any naturally occurring amino acid
<400> 5
   Gly Lys Asp Gln Ala Asp Lys His Val Xaa Val Glu His Ala Lys
   1               5                   10                  15
<210> 6
   <211> 489
   <212> DNA
   <213> Neolentinus lepideus
<400> 6
   atgagccacg aaggagcaac gtcagaagag ttcaagcaga ttgaagggaa gcggttcaag      60
   tacacgtatg gtttgggatg gacgtatgag atgtacttcc gctccttaac gcgatgtgta     120
   taccggattc tttcggggcc tttggcgggt agagttaact tccagcatgc gcactaccag     180
   aaaatcagag acaacgtctg gcaatgcagc tggctcgaag aaaccgggac agtagtctcc     240
   atggttgtcg actttgatca acagactgtc aagactttcg caacgttctc gagaggccac     300
   tgggatctcc ccgatcaggc gaaagggtgg aagcgcaatc cggaggacat ggcaagatgg     360
   aggactctcg cgcagaaagg aaaggaccag gcagacaagc atgtgctagt cgaacatgcg     420
   aaaatgtccg agttgacgag cgggcaggga gatctgccag acatagacga ttcttgggag     480
   accatgtag                                                             489

## Description

La présente invention s'inscrit dans le domaine de la préparation de composés biosourcés à valeur ajoutée par voie microbiologique, notamment fongique. Plus particulièrement, l'invention concerne un procédé de préparation d'un composé vinylphénolique à partir d'un acide hydroxycinnamique précurseur issu d'un matériau d'origine végétale, plus précisément issu d'un tourteau d'oléagineux, ainsi qu'un procédé plus global de production d'un composé vinylphénolique à partir d'un tourteau d'oléagineux.

Les tourteaux d'oléagineux sont les co-produits solides des procédés d'extraction de l'huile à partir des graines d'oléagineux, telles que les graines de colza, de tournesol ou de soja. Ils sont principalement composés de protéines, de matériaux cellulosiques et de minéraux. Le colza et le tournesol sont deux des plus importantes plantes oléagineuses cultivées dans le monde. La production mondiale de tourteaux de colza (RSM) et de tourteaux de tournesol (SFM) a été estimée pour l'année 2013 à respectivement 35,8 et 16,7 millions de tonnes. L'utilisation et la valorisation de ces tourteaux s'avèrent, de ce fait, d'un intérêt économique important, et ont fait l'objet de nombreuses études. Les tourteaux de colza et les tourteaux de tournesol ont ainsi été utilisés comme source de protéines pour l'alimentation animale ou pour amender les sols.

Plus récemment, ont émergé de nouvelles alternatives à l'usage traditionnel des tourteaux d'oléagineux en alimentation animale, en particulier pour la production de composés à valeur ajoutée d'intérêt biotechnologique et industriel. On peut notamment citer à titre d'exemple la transformation des tourteaux par voie chimique en matériaux naturels composites et plastiques et en additifs de synthèse ; ou encore, dans le domaine microbiologique, leur mise en oeuvre en tant que substrats physiques de croissance microbienne, tirant profit de leur structure fibreuse poreuse et de leur composition chimique adéquate constituant une source de carbone et d'azote, et riche en minéraux et vitamines. Les tourteaux d'oléagineux peuvent ainsi être utilisés soit en culture liquide, soit en fermentation solide, pour le développement de différents microorganismes, et en particulier des champignons filamenteux.

La structure et la composition des tourteaux d'oléagineux, en particulier des tourteaux de tournesol et des tourteaux de colza, offre en outre des perspectives intéressantes pour la production de molécules plateformes biosourcées d'intérêt industriel.

Ainsi, il a été proposé par l'art antérieur divers bioprocédés de production de molécules à valeur ajoutée à partir de tourteaux d'oléagineux. A titre d'exemple, on peut citer, pour les tourteaux de colza, la production d'acide succinique par *Actinobacillus succinogenes* (Chen et al. 2011, Enzyme Microb Technol, 48: 339-344), ou d'acides gras polyinsaturés par *Rhodosporidium toruloides* (Kiran et al. 2013, Biores Technol, 129: 650-654) ; et, pour les tourteaux de tournesol, la production de polyhydroxyalcanoates par *Cupriavidus necator* (Kachrimanidou et al. 2014, Biores Technol, 172: 131-130) ou d'acide clavulanique par *Streptomyces clavuligenes* (Sircar et al. 1998, Process Biochem 33: 283-289).

Les tourteaux d'oléagineux sont notamment connus pour leur teneur élevée en composés phénoliques, notamment en acides hydroxycinnamiques, tels que l'acide sinapique, l'acide férulique, l'acide caféique, l'acide p-coumarique, etc., qui y sont présents sous forme d'ester. En particulier, les tourteaux de colza sont très riches en acide sinapique (acide 4-hydroxy-3,5-diméthoxycinnamique) qui y représente 90 à 95 % des composés phénoliques, sous la forme de l'ester sinapine. Les tourteaux de tournesol contiennent majoritairement de l'acide chlorogénique, ester d'acide caféique et de quinine, et de l'acide caféique.

Ces acides hydroxycinnamiques issus de la modification de la lignine contenue dans les tourteaux sont susceptibles de constituer des précurseurs de composés biosourcés à haute valeur ajoutée, tels que des arômes, antioxydants, molécules plateforme pour la chimie verte.

Par exemple, le canolol, ou 2,6-diméthoxy-4-vinylphénol, également nommé 4-vinylsyringol, susceptible d'être obtenu par décarboxylation de l'acide sinapique, est connu pour être un agent antioxydant naturel puissant. Il a notamment été reporté dans la littérature que son activité antioxydante est comparable à celle du γ-tocophérol (Galano et al. 2011, J Phys Chem, 115(26): 8590-6). Il présente en outre des propriétés anti-carcinogénèse et préventives contre les infections induites par des bactéries telles que *Helicobacter pylori*, et constitue un piégeur de radicaux alkylpéroxyle. Il s'agit également d'une molécule plateforme pour la préparation de composés biosourcés. Le canolol présente ainsi un intérêt pour de nombreuses applications dans des domaines divers tels que les domaines de l'alimentaire, de la pharmacie, de la cosmétique, ou encore de biopolymères.

Un intérêt important a en outre également émergé récemment pour le 4-vinylguaiacol, qui présente des applications notamment en tant qu'arôme, agent antioxydant, agent antimicrobien et agent anticancéreux. Ce composé vinylphénolique peut notamment être obtenu par décarboxylation de son précurseur l'acide férulique (acide 4-hydroxy-3-méthoxy cinnamique).

Il n'existe cependant à l'heure actuelle aucun procédé de production de canolol ou de 4-vinylguaiacol en quantités compatibles avec un procédé industriel.

Il a notamment été proposé par l'art antérieur de produire du canolol à partir de tourteaux de colza, par voie chimique, par réaction de décarboxylation induite par la chaleur ou les microondes en présence d'une base. A titre d'exemple de tel procédé, on peut citer le procédé décrit dans la publication de Pudel et al. (2014, Oilseeds Fats Crops Lipids, 21(1): 103), qui prévoit le chauffage à haute température des tourteaux de colza sur un lit fluidisé suivi d'une extraction avec du dioxyde de carbone supercritique. Ce procédé est cependant délicat à mettre en oeuvre, les conditions opératoires devant être réglées de manière très précise. En outre, il est coûteux, les rendements obtenus ne s'avèrent pas satisfaisants, et il entraine une dégradation irréversible des protéines du tourteau.

Il a autrement été proposé par l'art antérieur de réaliser la réaction de décarboxylation des acides hydroxycinnamiques par voie enzymatique, par des enzymes à activité décarboxylase d'acide phénolique (désignées par l'abréviation PAD) d'origine bactérienne.

On peut citer notamment les travaux de Hu et al. (2014, Appl Microbiol Biotechnol DOI 10.1007/s00253-014-6313-3), qui mettent en oeuvre une PAD de *Bacillus licheniformis* pour la décarboxylation d'acides hydroxycinnamiques tels que l'acide férulique et l'acide caféique. Cette enzyme présente également une activité, mais très faible, de décarboxylation de l'acide sinapique.

Il a également été proposé par Morley et al. (2013, Green Chem., 15: 3312-3317) de réaliser la décarboxylation d'acides hydroxycinnamiques par une enzyme dérivée par mutagénèse de la PAD de la souche *Bacillus pumilus* UI-670, connue pour être apte à décarboxyler l'acide férulique en 4-vinylguaiacol, mais être inactive vis-à-vis de l'acide sinapique. Cette enzyme nommée SAD par ces auteurs permet de produire du canolol à partir d'acide sinapique. Cependant, son activité de transformation de l'acide férulique en 4-vinylguaiacol est fortement réduite par rapport à l'enzyme native.

Ainsi, il subsiste un besoin pour un procédé de type enzymatique / microbiologique, permettant de préparer un composé vinylphénolique à partir de son précurseur acide hydroxycinnamique, qui puisse être mis en oeuvre de manière efficace pour tous les principaux acides hydroxycinnamiques pouvant être libérés à partir de tourteaux d'oléagineux. Plus généralement, il subsiste un besoin pour un procédé de production par voie entièrement microbiologique de molécules vinylphénoliques à haute valeur ajoutée, utilisables en chimie verte, à partir de tourteaux d'oléagineux, ressource naturelle abondante et peu coûteuse.

La présente invention vise à proposer de tels procédés.

Il a maintenant été découvert par les présents inventeurs que des microorganismes particuliers, plus particulièrement des champignons filamenteux de l'espèce *Neolentinus lepideus*, produisent des enzymes à activité de décarboxylation d'acides phénoliques (enzymes PAD, pour Phenolic Acid Decarboxylase) qui présentent, de manière native, sans qu'il soit besoin de les modifier par des techniques de bioingénierie, une forte activité de décarboxylation de l'ensemble des acides hydroxycinnamiques présents dans les tourteaux d'oléagineux, et notamment aussi bien l'acide sinapique que l'acide férulique, l'acide caféique ou l'acide p-coumarique. La biotransformation catalysée par ces enzymes PAD particulières peut être réalisée aussi bien par mise en présence de l'acide hydroxycinnamique avec des cellules de *Neolentinus lepideus* en culture, qu'avec l'enzyme isolée de ces cellules, ou avec toute souche d'un organisme, notamment d'un organisme hétérologue, génétiquement modifié pour la produire.

Ainsi, selon un premier aspect, il est proposé selon la présente invention un procédé de préparation d'un composé vinylphénolique, en particulier un composé 4-vinylphénolique, à partir d'un acide hydroxycinnamique précurseur, notamment un acide hydroxycinnamique issu d'un tourteau d'oléagineux, tel que l'acide sinapique, l'acide férulique, l'acide p-coumarique ou l'acide caféique. Ce procédé comprend une étape de biotransformation de l'acide hydroxycinnamique par mise en présence avec une enzyme à activité décarboxylase d'acide phénolique (enzyme PAD) d'un microorganisme de l'espèce *Neolentinus lepideus.*

Par mise en présence avec une enzyme à activité décarboxylase d'acide phénolique (enzyme PAD) d'un microorganisme de l'espèce *Neolentinus lepideus*, on englobe selon la présente invention aussi bien la mise en présence de l'acide hydroxycinnamique avec une telle enzyme PAD isolée des cellules qui l'ont produite, ou le cas échéant produite par synthèse chimique, que la mise en présence de l'acide hydroxycinnamique avec des cellules produisant cette enzyme en culture, notamment des cellules de l'espèce *Neolentinus lepideus*, ou tout autre cellule génétiquement modifiée pour produire une telle enzyme.

*Neolentinus lepideus* est un champignon filamenteux basidiomycète du genre *Neolentinus*, connu en lui-même notamment dans le domaine alimentaire.

Comme exposé ci-avant, il a été découvert par les présents inventeurs que l'enzyme PAD native de ce champignon de l'espèce *Neolentinus lepideus* est capable de convertir l'acide sinapique en canolol avec un rendement élevé, d'environ 75 %, les taux de canolol obtenus étant supérieurs à 1 g/l, mais aussi notamment, avec un rendement également élevé, l'acide férulique en 4-vinylguaiacol, l'acide *p*-coumarique en 4-vinylphénol et l'acide caféique en 4-vinylcatéchol.

Les enzymes PAD sont des enzymes intracellulaires connues en elles-mêmes, de petite taille (161 à 178 acides aminés), actives sous forme de dimères et ne nécessitant pas de cofacteur. Le pourcentage de similarité entre les PAD d'origine bactérienne et les PAD d'origine fongique est très faible, de l'ordre de 20 %. Seule la zone N-terminale de la protéine est relativement bien conservée.

La séquence de la PAD d'une souche de *Neolentinus lepideus* a récemment été publiée sur le portail du DOE Join Genome Institute (http://genome.jgi.doe.gov/Neole1/Neole1.home.html; protein id: 1126845, SEQ ID NO: 1). Il a été découvert par les présents inventeurs que cette PAD, de même que les PAD des autres souches de *Neolentinus lepideus*, dans la mesure où la séquence en acides aminés de l'enzyme PAD est fortement conservée chez l'espèce *Neolentinus lepideus*, permet de réaliser la décarboxylation de l'ensemble des acides hydroxycinnamiques présents dans les tourteaux d'oléagineux avec un rendement important.

Dans des modes de mise en œuvre particuliers de l'invention, l'enzyme à activité décarboxylase d'acide phénolique présente au moins 90 %, de préférence au moins 95 %, d'identité en amino acides avec la séquence SEQ ID NO: 1.

L'enzyme à activité décarboxylase d'acide phénolique peut présenter, par rapport à la séquence SEQ ID NO: 1, une ou plusieurs délétion(s), addition(s) et/ou substitution(s) d'un ou plusieurs acides aminés, qui ne modifient pas l'activité de biotransformation des acides hydroxycinnamiques en composés vinylphénoliques.

Il est du ressort de l'homme du métier de déterminer quelles modifications peuvent être apportées à la séquence SEQ ID NO: 1, sans altérer de manière significative l'activité de biotransformation des acides hydroxycinnamiques, à partir de ses connaissances théoriques et/ou de tests expérimentaux.

Entrent par exemple dans le cadre de l'invention des enzymes obtenues par modifications de la séquence SEQ ID NO: 1 par substitution d'acides aminés par des acides aminés de même famille, par exemple d'un résidu basique tel que l'arginine par un autre résidu basique comme un résidu lysine, d'un résidu acide tel que l'aspartate par un autre résidu acide comme le glutamate, d'un résidu polaire comme la sérine par un autre résidu polaire comme la thréonine, d'un résidu aliphatique comme la leucine par un autre résidu aliphatique comme l'isoleucine, etc.

En particulier, l'enzyme à activité décarboxylase contient de préférence au moins une, de préférence plusieurs, des séquences SEQ ID NO: 2, SEQ ID NO: 3, SEQ ID NO: 4 et SEQ ID NO: 5 suivantes :
SEQ ID NO: 2 - Ser His Glu Gly Ala Thr Ser Glu Glu Phe Lys Gln Ile Glu Gly Lys
SEQ ID NO: 3 - Ile Xaa Ser Gly Pro Xaa Ala Gly Arg
SEQ ID NO: 4 - Val Val Ser Met Val Val Asp Phe Asp Gln Gln Thr Val Lys Thr Phe Ala Thr Phe Ser Arg Gly His Trp Asp Xaa Pro Asp Gln Ala Lys
SEQ ID NO: 5 - Gly Lys Asp Gln Ala Asp Lys His Val Xaa Val Glu His Ala Lys
dans chacune desquelles Xaa représente un quelconque acide aminé naturel.

En particulier, dans la séquence SEQ ID NO: 3, les deux résidus Xaa peuvent être identiques ou différents.

De préférence, dans au moins une des séquences SEQ ID NO: 3, SEQ ID NO: 4 et SEQ ID NO: 5, de préférence dans deux de ces séquences, et préférentiellement dans l'ensemble de ces séquences, Xaa (ou au moins un des résidus Xaa pour la séquence SEQ ID NO: 3, de préférence les deux résidus Xaa) représente un résidu leucine ou un résidu isoleucine.

Préférentiellement, dans au moins une des séquences SEQ ID NO: 3, SEQ ID NO: 4 et SEQ ID NO: 5, de préférence dans deux de ces séquences, et préférentiellement dans l'ensemble de ces séquences, Xaa (ou au moins un des résidus Xaa pour la séquence SEQ ID NO: 3, de préférence les deux résidus Xaa) est un résidu isoleucine.

L'étape de biotransformation de l'acide hydroxycinnamique peut notamment être réalisée par culture de cellules du microorganisme de l'espèce *Neolentinus lepideus* en présence de l'acide hydroxycinnamique précurseur à transformer.

En particulier, il peut être mis en œuvre la souche de *Neolentinus lepideus* déposée sous le numéro ATCC 36557 à l'American Type Culture Collection (ATCC) ou encore déposée dans la collection CIRM-BRFM (International Centre of Microbial Resources-Banque de Ressources Fongiques de Marseille, INRA, Marseille, France)), sous le numéro BRFM 15. Cette souche s'avère particulièrement efficace pour réaliser la biotransformation des acides hydroxycinnamiques tels que l'acide sinapique, l'acide férulique, l'acide p-coumarique et l'acide caféique, en les dérivés vinylphénoliques correspondants. Les rendements molaires qu'elle permet d'obtenir sont aussi élevés que 70 %. La PAD de cette souche contient notamment les séquences SEQ ID NO: 2, SEQ ID NO: 3, SEQ ID NO: 4 et SEQ ID NO: 5, dans lesquelles le résidu Xaa représente un résidu isoleucine.

Dans des modes de mise en œuvre particuliers de l'invention, il est mis en œuvre la souche de *Neolentinus lepideus* HHB14362-Sp. déposée dans la collection du Center for Forest Mycology Research Culture Collection (Madison, USA). La PAD de cette souche présente la séquence SEQ ID NO: 1.

La culture des cellules est réalisée dans des conditions classiques en elles-mêmes, dans un milieu de culture contenant les constituants nécessaires à la survie des cellules.

L'étape de biotransformation de l'acide hydroxycinnamique peut autrement être réalisée par mise en présence de l'acide hydroxycinnamique précurseur à transformer avec l'enzyme PAD purifiée, issue d'une souche sauvage de *Neolentinus lepideus* ou d'une production par un organisme hétérologue, tel qu'*Aspergillus niger* par exemple, réalisée de manière classique en elle-même pour l'homme du métier.

L'enzyme isolée peut alors notamment être mise en œuvre sous forme immobilisée sur support solide. Il est du ressort de l'homme du métier de déterminer les conditions opératoires adéquates pour assurer une bonne activité de l'enzyme.

Dans des modes de mise en œuvre particuliers de l'invention, la concentration en acide hydroxycinnamique précurseur dans le milieu réactionnel, notamment dans le milieu de culture cellulaire, est comprise entre 100 et 400 mg/l, de préférence comprise entre 300 et 400 mg/l.

L'étape de biotransformation de l'acide hydroxycinnamique par mise en présence avec l'enzyme PAD du microorganisme de l'espèce *Neolentinus lepideus* est de préférence réalisée à une température comprise entre environ 37 °C et environ 45 °C.

Les rendements de réaction peuvent en outre être accrus en séparant le composé vinylphénolique du milieu réactionnel, par exemple du milieu de culture cellulaire, à l'issue de la réaction, au fur et à mesure de sa formation. Ceci peut notamment être réalisé par mise en œuvre de résines aptes à l'adsorber spécifiquement, mais n'adsorbant pas ou peu les acides hydroxycinnamiques, par exemple des copolymères de styrène et de divinylbenzène, tels que les résines commercialisées sous les noms HP20 et XAD2.

Ainsi, dans des modes de mise en œuvre particuliers de l'invention, l'étape de biotransformation de l'acide hydroxycinnamique précurseur est réalisée en présence dans le milieu réactionnel d'une résine apte à adsorber le composé vinylphénolique formé au fur et à mesure de sa production.

Une telle caractéristique permet avantageusement d'éviter la dégradation du composé vinylphénolique formé, de limiter sa toxicité éventuelle vis-à-vis des microorganismes fongiques, et d'augmenter la quantité de composé produite.

Dans des modes de mise en œuvre particuliers de l'invention, l'étape de biotransformation de l'acide hydroxycinnamique précurseur est réalisée dans un système biphasique, plus précisément un mélange d'eau et d'un solvant organique, tel que l'hexane. Le solvant organique est choisi de sorte à ne pas inactiver l'enzyme PAD. Dans les modes de mise en œuvre de l'invention dans lesquels l'étape de biotransformation de l'acide hydroxycinnamique précurseur est réalisée par mise en présence avec des cellules de *Neolentinus lepideus* en culture, le solvant organique est en outre choisi de sorte à ne pas présenter de toxicité vis-à-vis de ces cellules.

Une telle caractéristique permet avantageusement d'augmenter le rendement de la réaction de biotransformation, par capture du composé vinylphénolique par le solvant organique au fur et à mesure de sa formation dans le milieu réactionnel.

L'acide hydroxycinnamique précurseur peut notamment être apporté dans le milieu réactionnel sous forme d'une solution aqueuse. Il peut autrement être apporté dans le milieu réactionnel en solution dans un solvant organique, en particulier dans un alcool, par exemple dans l'éthanol. Une telle caractéristique permet avantageusement de diminuer la quantité de produits aromatiques secondaires, autres que le vinylphénol, formée au cours de la réaction de biotransformation.

Selon l'invention, l'acide hydroxycinnamique et le composé vinylphénolique peuvent consister en, respectivement :
- l'acide sinapique et le canolol, de formules respectives (Ia) et (IIa) : 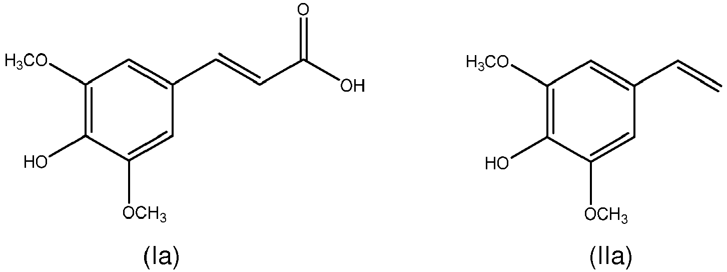
- l'acide caféique et le 4-vinylcatéchol, de formules respectives (Ib) et (IIb) : 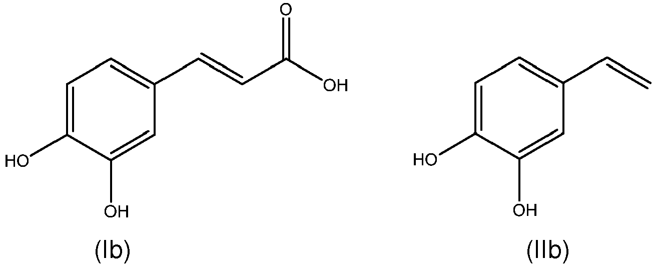
- l'acide p-coumarique et le 4-vinylphénol, de formules respectives (Ic) et (IIc) : 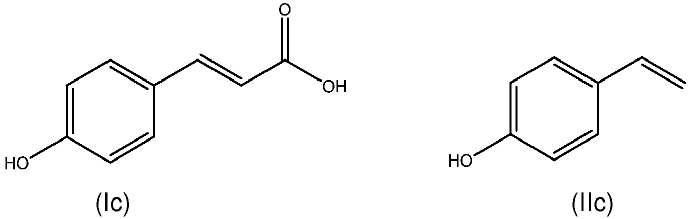
- ou l'acide férulique et le 4-vinylguaiacol, de formules respectives (Id) et (IId) :

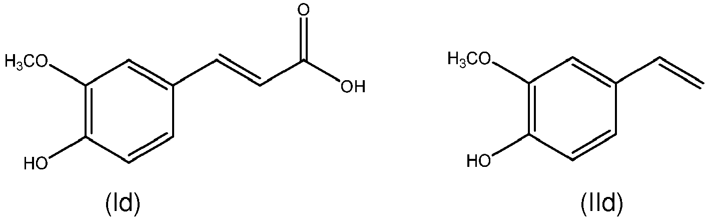

Selon un autre aspect, la présente invention concerne un procédé de production d'un composé vinylphénolique à partir d'un tourteau d'oléagineux, comprenant :
- une étape de libération, du tourteau d'oléagineux, d'un acide hydroxycinnamique précurseur du composé vinylphénolique,
- et une étape de transformation de l'acide hydroxycinnamique ainsi libéré en le composé vinylphénolique par un procédé de préparation selon l'invention, répondant à l'une ou plusieurs des caractéristiques ci-avant.

Ces deux étapes peuvent être séparées par une étape de concentration de l'acide hydroxycinnamique, par toute méthode classique en elle-même pour l'homme du métier.

Dans des modes de mises en œuvre particuliers de l'invention, l'oléagineux est choisi parmi le colza, le tournesol et le soja. Les tourteaux correspondants, en particulier les tourteaux de colza et les tourteaux de tournesol, sont avantageusement disponibles en grande quantité et peu coûteux.

On entend dans la présente description, par tourteau d'oléagineux, le tourteau en lui-même ou toute fraction d'un tel tourteau.

Les acides hydroxycinnamiques y sont présents sous forme d'ester. Par exemple, l'acide sinapique est présent dans les tourteaux du colza sous forme de sinapine, également nommée choline ester d'acide sinapique.

L'étape de libération de l'acide hydroxycinnamique du tourteau d'oléagineux peut être réalisée par voie chimique.

Autrement, cette étape de libération de l'acide hydroxycinnamique du tourteau d'oléagineux peut être réalisée par mise en présence du tourteau d'oléagineux avec une enzyme de type cinnamoyl esterase, telle qu'une féruloyl esterase A (Fae A), une féruloyl esterase B (Fae B), ou une chlorogénate esterase (Chlo E), d'un microorganisme de l'espèce *Aspergillus niger,* dans des conditions permettant la libération de l'acide hydroxycinnamique du tourteau d'oléagineux. Il entre dans les compétences de l'homme du métier de déterminer ces conditions, en fonction de l'enzyme particulière mise en œuvre.

Par exemple, en particulier lorsque le tourteau d'oléagineux est un tourteau de colza, l'acide hydroxycinnamique est l'acide sinapique, et que l'étape de libération de cet acide sinapique est réalisée par mise en présence du tourteau de colza avec l'enzyme FaeA, la mise en présence du tourteau d'oléagineux avec l'enzyme est réalisée selon au moins l'une, de préférence plusieurs, des conditions suivantes : température comprise entre 45 et 55 °C, temps de mise en présence d'au moins 3 heures, et quantité d'enzyme de type cinnamoyl esterase comprise entre 26 et 40 nkat par gramme de tourteau. De telles conditions permettent avantageusement d'obtenir les meilleurs rendements de libération de l'acide hydroxycinnamique du tourteau d'oléagineux.

L'étape de libération de l'acide hydroxycinnamique du tourteau d'oléagineux peut notamment être réalisée par mise en présence du tourteau avec une enzyme de type cinnamoyl esterase purifiée, issue d'une souche sauvage d'*Aspergillus niger* ou d'une production par un autre organisme hétérologue, réalisée de manière classique en elle-même pour l'homme du métier ; ou d'un mélange de telles enzymes purifiées.

Dans des modes de mise en œuvre particuliers de l'invention, l'étape de libération de l'acide hydroxycinnamique peut autrement être réalisée par mise en présence du tourteau d'oléagineux avec une préparation enzymatique issue du surnageant d'une culture de cellules du microorganisme de l'espèce *Aspergillus niger* en présence d'un substrat naturel choisi parmi les tourteaux d'oléagineux et la pulpe de betterave sucrière.

En particulier, il a été découvert par les présents inventeurs qu'il était possible, à partir de cultures d'*Aspergillus niger* réalisées de manière adéquate sur des substrats inducteurs naturels appropriés, plus particulièrement des tourteaux d'oléagineux ou de la pulpe de betterave sucrière, d'obtenir une préparation enzymatique présentant un spectre d'activités enzymatiques nécessaires à la libération d'acide hydroxycinnamique, notamment d'acide sinapique, à partir de tourteaux d'oléagineux.

Le substrat naturel mis en œuvre, qui joue le rôle de source de carbone naturelle et d'inducteur d'enzyme, peut notamment être choisi de sorte à permettre la production majoritaire, par le microorganisme de l'espèce *Aspergillus niger,* d'une enzyme de type cinnamoyl esterase particulière parmi l'ensemble des enzymes de type cinnamoyl esterase susceptibles d'être produites, et notamment de l'enzyme qui présente la plus forte activité pour réaliser la libération de l'acide hydroxycinnamique particulier visé.

Par exemple, cultivées en présence d'un tourteau de colza en tant que substrat carboné naturel, les cellules de l'espèce *Aspergillus niger* produiront majoritairement, dans le surnageant de culture, la féruloyl esterase A (Fae A), particulièrement active par exemple pour la libération d'acide sinapique des tourteaux de colza, alors que cultivées en présence de tourteau de tournesol, les cellules de l'espèce *Aspergillus niger* produiront majoritairement la féruloyl esterase B (Fae B).

Le surnageant de la culture de cellules du microorganisme de l'espèce *Aspergillus niger* en présence du substrat naturel peut notamment être prélevé après au moins 4 jours, préférentiellement après environ 10 jours, de culture cellulaire.

La préparation enzymatique, issue du surnageant de la culture de cellules du microorganisme de l'espèce *Aspergillus niger* en présence d'un substrat naturel, peut aussi bien consister en ce surnageant en lui-même, qu'en un concentré de ce surnageant, obtenu par exemple par ultrafiltration ou par lyophilisation, ou une fraction de ce surnageant.

Dans des modes de mise en œuvre particuliers de l'invention, il est mis en œuvre, pour l'étape de libération de l'acide hydroxycinnamique du tourteau d'oléagineux, une préparation enzymatique produite par la souche CNCM I-1472 *d'Aspergillus niger* (déposée le 31 août 1994 auprès de la Collection Nationale de Cultures de Micro-organismes, Paris, France ; et également déposée dans la collection CIRM-BRFM (International Centre of Microbial Resources-Banque de Ressources Fongiques de Marseille, INRA, Marseille, France) sous le n° BRFM 281).

En présence de substrats naturels inducteurs tels que le tourteau de colza ou le tourteau de tournesol, la souche d'*A*. *niger* BRFM 281 est capable de produire les cinnamoyl estérases : féruloyl estérase A (Fae A), féruloyl estérase B (Fae B) et/ou chlorogénate estérase (Chlo E). Ces enzymes permettent l'hydrolyse d'esters d'acides cinnamiques.

Le procédé de production d'un composé vinylphénolique, à partir d'un tourteau d'oléagineux, selon la présente invention, mettant en œuvre une préparation enzymatique pour l'étape de libération de l'acide hydroxycinnamique du tourteau d'oléagineux, présente en particulier l'avantage d'être entièrement de nature biotechnologique, et de ne mettre en œuvre aucun réactif chimique.

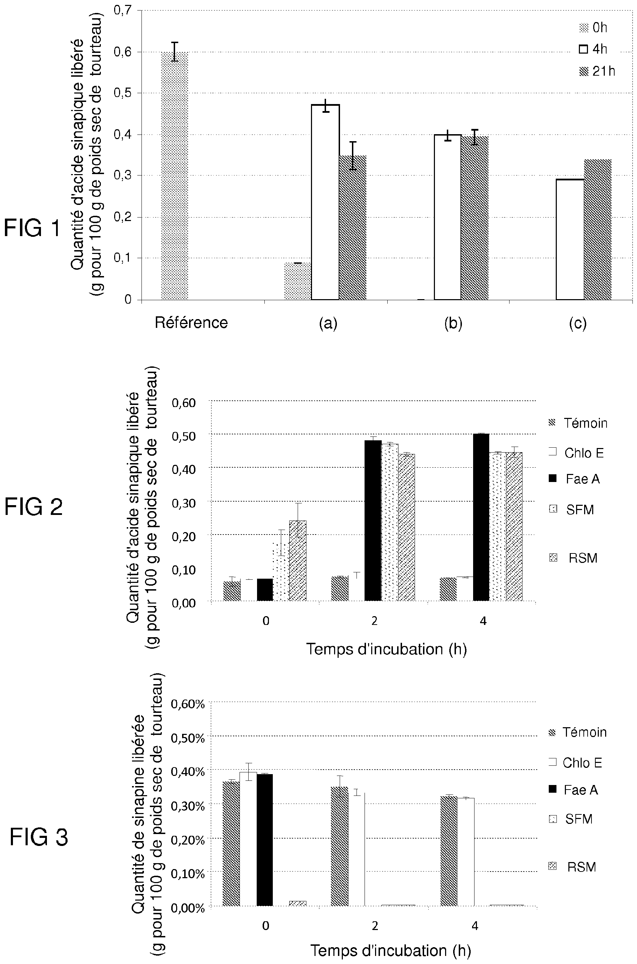

Les caractéristiques et avantages des procédés selon l'invention apparaîtront plus clairement à la lumière des exemples de mise en œuvre ci-après, fournis à simple titre illustratif et nullement limitatifs de l'invention, avec l'appui des figures 1 à 13, dans lesquelles :
- la figure 1 représente un graphe illustrant la quantité d'acide sinapique libéré à partir de tourteau de colza conformément à l'invention, par un procédé mettant en œuvre une préparation enzymatique obtenue par culture de la souche *Aspergillus niger* BRFM 281 sur (a) tourteau de colza, (b) tourteau de tournesol, (c) pulpe de betterave sucrière, pour différents temps d'incubation du tourteau de colza avec ladite préparation enzymatique : 0, 4 et 21 h ; la référence correspondant à l'hydrolyse alcaline de tourteau de colza ;
- la figure 2 représente un graphe illustrant la quantité d'acide sinapique libéré à partir de tourteau de colza conformément à l'invention, après différents temps d'incubation, par un procédé mettant en œuvre l'enzyme Chlo E pure, l'enzyme Fae A pure, une préparation enzymatique obtenue par culture de la souche *Aspergillus niger* BRFM 281 sur tourteau de tournesol (SFM), ou une préparation enzymatique obtenue par culture de la souche *Aspergillus niger* BRFM 281 sur tourteau de colza (RSM) ; le témoin correspondant à une réaction dans le tampon en l'absence d'enzyme ;
- la figure 3 représente un graphe illustrant, en fonction du temps d'incubation, la quantité de sinapine libérée pour un tourteau de colza mis en présence, conformément à l'invention, avec l'enzyme Chlo E pure, l'enzyme Fae A pure, une préparation enzymatique obtenue par culture de la souche *Aspergillus niger* BRFM 281 sur tourteau de tournesol (SFM), ou une préparation enzymatique obtenue par culture de la souche *Aspergillus niger* BRFM 281 sur tourteau de colza (RSM) ; le témoin correspondant à une réaction dans le tampon en l'absence d'enzyme ;

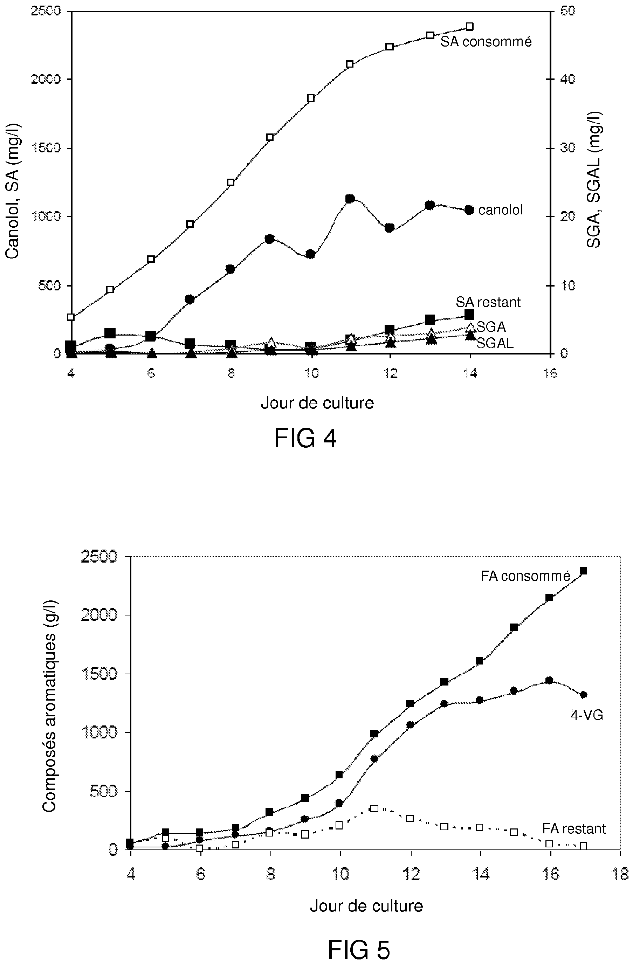

- la figure 4 représente un graphe montrant les concentrations d'acide sinapique (SA) restant et consommé, et de canolol formé, dans le milieu de culture de la souche *N. lepideus* BRFM 15 supplémenté en acide sinapique lors de la mise en œuvre d'un procédé conforme à l'invention de biotransformation d'acide sinapique en canolol ; sont également indiquées sur le graphe les concentrations en acide syringique (SGA) et en syringaldéhyde (SGAL) formés dans le milieu de culture ;
- la figure 5 représente un graphe montrant les concentrations d'acide férulique (FA) restant et consommé, et de 4-vinylguaiacol formé, dans le milieu de culture de la souche *N. lepideus* BRFM 15 supplémenté en acide férulique lors de la mise en œuvre d'un procédé conforme à l'invention de biotransformation d'acide férulique en 4-vinylguaiacol (4-VG) ;

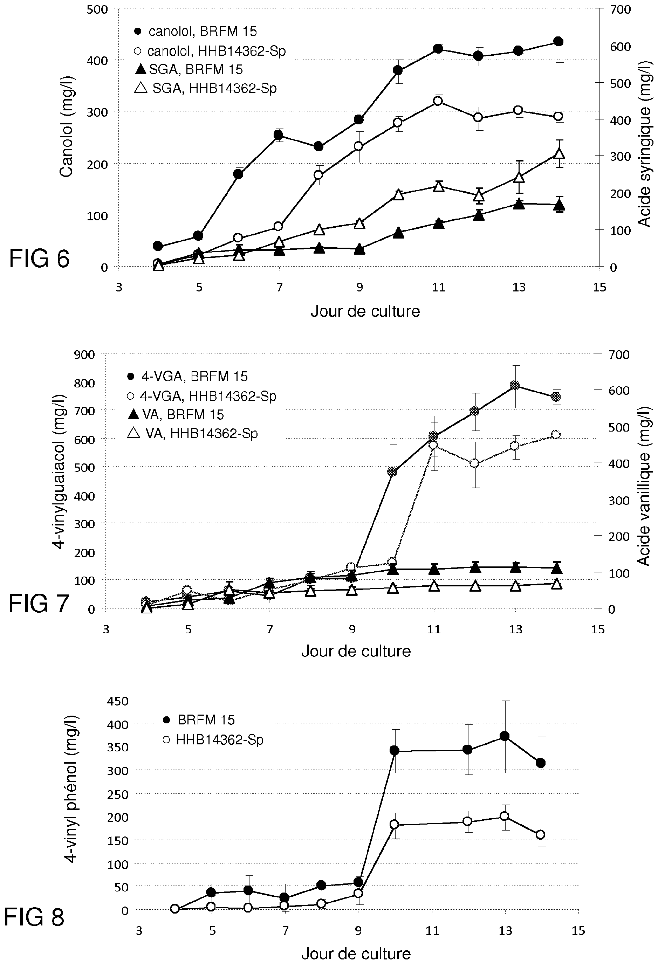

- la figure 6 représente un graphe montrant les concentrations de canolol formé, dans les milieux de culture respectivement de la souche *N. lepideus* BRFM 15 et de la souche *N. lepideus* HHB14362-Sp, supplémentés en acide sinapique lors de la mise en œuvre d'un procédé conforme à l'invention de biotransformation d'acide sinapique en canolol ; sont également indiquées sur le graphe les concentrations en acide syringique (SGA) formé dans le milieu de culture ;
- la figure 7 représente un graphe montrant les concentrations de 4-vinylguaiacol (4-VGA) formé, dans les milieux de culture respectivement de la souche *N. lepideus* BRFM 15 et de la de la souche *N. lepideus* HHB14362-Sp, supplémentés en acide férulique lors de la mise en œuvre d'un procédé conforme à l'invention de biotransformation d'acide férulique en 4-vinylguaiacol ; sont également indiquées sur le graphe les concentrations en acide vanillique (VA) formé dans le milieu de culture ;
- la figure 8 représente un graphe montrant les concentrations de 4-vinylphénol (4-VP) formé, dans les milieux de culture respectivement de la souche *N. lepideus* BRFM 15 et de la souche *N. lepideus* HHB14362-Sp, supplémentés en acide *p*-coumarique lors de la mise en œuvre d'un procédé conforme à l'invention de biotransformation d'acide *p*-coumarique en 4-vinylphénol ;

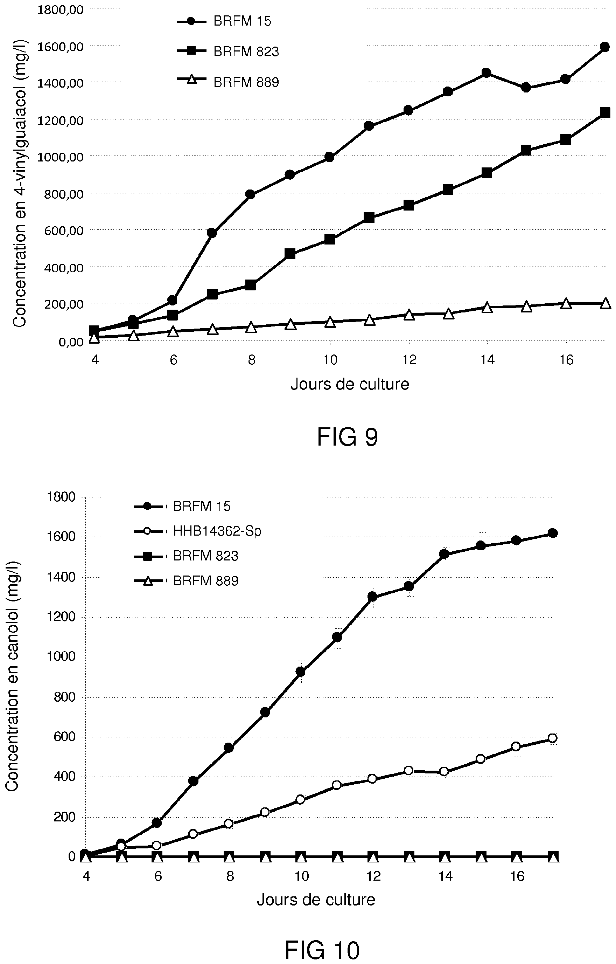

- la figure 9 représente un graphe montrant les concentrations de 4-vinylguaiacol (4-VGA) formé, dans les milieux de culture respectivement de la souche *N. lepideus* BRFM 15, de la souche *S. commune* BRFM 823 et de la souche *S. hirsutum* BRFM 889, supplémentés en acide férulique lors de la mise en œuvre d'un procédé conforme à l'invention de biotransformation d'acide férulique en 4-vinylguaiacol ;
- la figure 10 représente un graphe montrant les concentrations de canolol formé, dans les milieux de culture respectivement de la souche *N. lepideus* BRFM 15, de la souche *N. lepideus* HHB14362-Sp, de la souche S. *commune* BRFM 823 et de la souche *S. hirsutum* BRFM 889, supplémentés en acide sinapique lors de la mise en œuvre d'un procédé conforme à l'invention de biotransformation d'acide sinapique en canolol ;

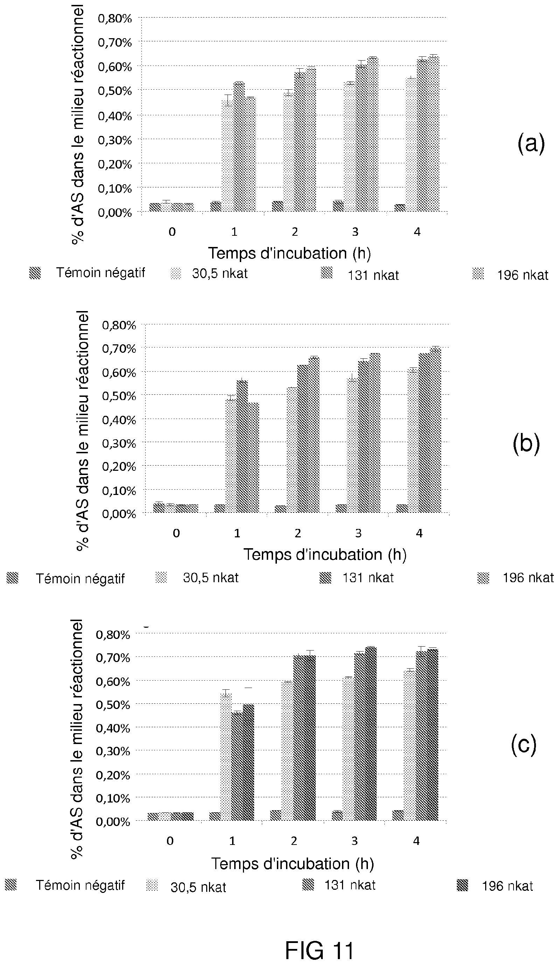

- la figure 11 représente des graphes montrant le % d'acide sinapique (AS) libéré dans le milieu réactionnel à partir de tourteau de colza conformément à l'invention, au moyen de l'enzyme FaeA *d'Aspergillus niger,* en fonction du temps d'incubation avec l'enzyme, ce pourcentage étant exprimé en g d'd'AS pour 100 g de tourteau initial (matière sèche), à différentes concentrations d'enzyme dans le milieu réactionnel (30,5, 131 ou 196 nkat), aux températures de (a) 37°C, (b) 45°C, (c) 55°C; le témoin négatif correspondant au milieu sans enzyme ;

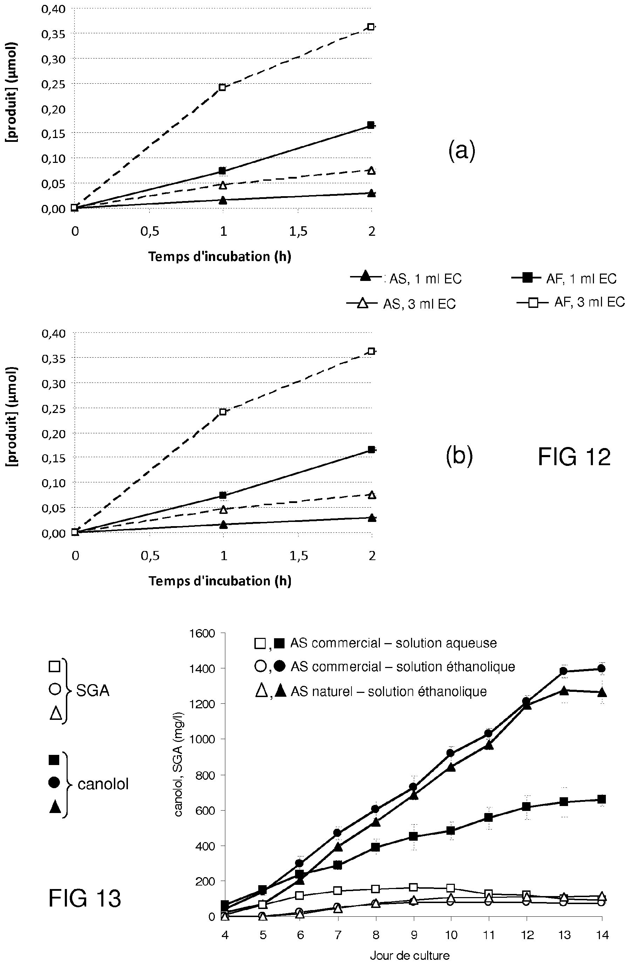

- la figure 12 représente des graphes montrant la concentration de vinylguaiacol ou canolol ([produit]) obtenu en fonction du temps d'incubation d'acides hydroxycinnamiques (acide sinapique AS ou acide férulique AF) avec un extrait intracellulaire brut (EC) de la souche *N. lepideus* BRFM 15 conformément à l'invention, pour différents volumes d'extrait intracellulaire EC mis en œuvre (1 ml, 3 ml), et aux températures : (a) 30 °C, (b) 37 °C ;
- et la figure 13 représente un graphe montrant les concentrations de canolol et d'acide syringique (SGA) obtenus en fonction du temps d'incubation d'acide sinapique (AS) avec des cellules de la souche N. *lepideus* BRFM 15 conformément à l'invention, dans des conditions dans lesquelles l'acide sinapique est d'origine naturelle (issu de l'hydrolyse du tourteau de colza par l'enzyme Fae A conformément à l'invention) ou commerciale, et apporté en solution aqueuse ou éthanolique ; sur cette figure, les symboles pleins représentent le canolol, et les symboles vides représentent l'acide syringique (SGA).

### A/ Matériel et méthodes

### Matériel végétal

Les tourteaux de colza et de tournesol ont été fournis par le Centre Technique Interprofessionnel des Oléagineux et du Chanvre (CETIOM, Pessac, France, désormais dénommé Terreslnovia). Avant leur utilisation en tant que substrat de croissance fongique, les tourteaux ont été broyés pour obtenir des particules de taille comprise entre 0,18 and 0,8 mm. Deux lots différents de chacun des types de tourteaux, variant dans l'origine géographique et le mode de broyage, ont été utilisés et nommés comme suit : SFM1 et SFM3 pour les tourteaux de tournesol ; RSM2 et RSM4 pour les tourteaux de colza. SFM1 et RSM2 ont été obtenus à 105 ± 2°C pendant 50 ± 5 min; SFM3 et RSM4 ont été obtenus à 107 ± 2°C pendant 80 ± 5 min.

### Produits chimiques

Tous les produits chimiques ont été obtenus auprès de Sigma-Aldrich.

### Microorganismes et conditions de culture

Les souches *N. lepideus* ATCC 36557 (BRFM 15) et *Aspergillus niger* I-1472 (BRFM 281) étudiées ont été déposées dans la collection CIRM-BRFM (International Centre of Microbial Resources-Banque de Ressources Fongiques de Marseille, INRA, Marseille, France). La souche *N. lepideus* HHB14362-Sp. a été déposée dans la collection du Center for Forest Mycology Research Culture Collection (Madison, USA).

Ces souches sont conservées sur gélose en pente à 4 °C.

Pour les tests de bioconversion d'acide hydroxycinnamique en composés vinylphénoliques, la souche *N. lepideus* (BRFM 15 ou HHB14362-Sp.) a été cultivée sur le milieu de préculture et de culture suivant : glucose (20 g.l⁻¹), extrait de levure (0,5 g.l⁻¹), diammonium tartrate (1,84 g.l⁻¹), KH₂PO₄ (0,2 g.l⁻¹), CaCl₂·2H₂O (0,00132 g.l⁻¹), MgSO₄·7H₂O (0,5 g.l⁻¹), et thiamine chlorhydrate (0,0025 g.l⁻¹). L'inoculum a été préparé à partir de précultures de 10 jours à 30 °C dans des fioles de Roux contenant 180 ml de milieu.

Les cultures ont été réalisées à 30 °C et 120 tr/mn dans des fioles d'Erlenmeyer bafflées de 250 ml contenant 100 ml de milieu. Après 3 jours d'incubation, l'acide sinapique, l'acide férulique, l'acide p-coumarique ou l'acide caféique (précurseur phénolique) est ajouté sous forme d'une solution stérilisée par filtration, à une concentration finale déterminée, par exemple de 0,3 g.l⁻¹.

L'alimentation en précurseur phénolique a été réalisée quotidiennement de sorte à maintenir la concentration finale déterminée, par exemple 0,3 g.l⁻¹, dans le milieu de culture. Dans le cas de cultures réalisées en présence d'une résine adsorbante HP20, l'adsorbant (100 g.l⁻¹) a été ajouté directement sous forme de particules libres au jour 4 de culture. Ensuite, aux jours 7, 10 et 14 de culture, le milieu de culture et la résine ont été éliminés des flacons et séparés par filtration. Les métabolites adsorbés sur la résine ont été extraits deux fois avec de l'éthanol pur, par agitation pendant 2 h à 200-300 tr/min avec un agitateur magnétique, et analysé par HPLC, ainsi que les composés restant dans le milieu de culture.

### Méthodes analytiques

Le contenu en matière sèches des tourteaux a été réalisé par séchage jusqu'à obtenir une masse constante à 110 °C.

L'analyse par chromatographie liquide haute performance (HPLC) a été réalisée à 220 nm et 30 °C sur un modèle Agilent 1100 équipé d'un détecteur UV/Vis variable et d'un injecteur automatique. Les séparations ont été réalisées sur colonne C30 phase inverse (YMC® Carotenoid 3 *µ*m, 4,6 x 150 mm, Waters, à un débit de 0,8 ml.min⁻¹, avec pour phases mobiles : 0,05% acide phosphorique dans l'eau (solvant A) et acétonitrile 100% (solvant B). Le gradient a été fixé comme suit: solvant B à 15% pendant 5 min, augmenté jusqu'à 40% en 15 min, puis jusqu'à 100% en 5 min jusqu'à la fin (28 min). Les données ont été traitées par la Agilent 1100 ChemStation, et la quantification a été réalisée par calibration standard externe.

### B/ Production de canolol

### B.1/ Libération d'acide sinapique de tourteaux de colza par un bouillon de culture d'Asperaillus niger

Les cultures sont réalisées dans des fioles d'Erlenmeyer bafflées de 500 ml, avec 100 ml de milieu contenant, par litre : 1,842 g de tartrate de diammonium en tant que source d'azote, 0,5 g d'extrait de levure, 0,2 g de KH₂PO₄, 0,0132 g de CaCl₂·2H₂O et 0,5 g de MgSO₄·7H₂O. Les substrats naturels, tourteau de colza, tourteau de tournesol ou pulpe de betterave sucrière (fraction 0,18-0,8 mm) à une concentration de 15 g/l sont utilisés en tant que source de carbone naturel et inducteur d'enzyme. 2,5 g/l de maltose sont utilisés en tant qu'activateur de croissance. Les cultures sont inoculées avec 2.10⁵ conidiospores d'*A*. *niger* par ml. Les cultures sont réalisées à 30 °C et 105 tr/min.

### Analyse de l'activité des enzymes présentes dans le bouillon de culture

Le suivi de l'activité des enzymes Fae A, Fae B et Chlo E est suivi pendant 11 jours (J1 à J11) d'incubation à 30 °C et105 tr/min.

Le dosage des activités enzymatiques est réalisé de manière classique en elle-même, en utilisant en tant que substrat le méthyl sinapate pour l'enzyme Fae A, le méthyl coumarate pour l'enzyme Fae B et l'acide chlorogénique pour l'enzyme Chlo E, selon les protocoles opératoires décrits dans les publications de Benoit et al. 2007, Applied and Environmental Microbiology 73: 5624-5632 ; et de Record et al. 2003, Applied Microbiology and Biotechnology 62: 349-355.

En présence de RSM4 comme substrat inducteur dans le milieu de culture, la souche d'*A*. *niger* BRFM 281 produit majoritairement de la Fae A, avec un pic d'activité au jour J10 (0,67 nkat/mL). Dans une moindre mesure, il est également produit de la Fae B (maximum 0,44 nkat/mL au jour J10) et de la Chlo E (maximum 0,30 nkat/mL au jour J10).

En présence de SFM1 comme substrat inducteur dans le milieu de culture, la souche d'*A*. *niger* BRFM 281 produit majoritairement de la Fae B, avec un pic d'activité au jour J10 (0,75 nkat/mL). Dans une moindre mesure, il est également produit de la Fae A (maximum 0,32 nkat/mL au jour J5) et de la Chlo E (maximum 0,24 nkat /mL au jour J8).

### Libération d'acide sinapique

Après 4 jours d'incubation à 30 °C et 105 tr/min, le mycélium est éliminé par filtration et le surnageant est filtré sur des unités de filtration de 0,22 µm.

Ensuite, le surnageant, c'est-à-dire le bouillon de culture dépourvu de cellules contenant les enzymes hydrolytiques (préparation enzymatique), est utilisé pour réaliser la libération d'acide sinapique de tourteaux de colza, comme suit: 100 ml de surnageant est incubé à 30 °C et 115 tr/min avec 5 g de tourteau de colza RSM4 (fraction 0,18-0,8 mm). La libération de l'acide sinapique depuis le tourteau est mesurée par analyse HPLC, selon la méthode indiquée ci-avant, après respectivement 0, 4 et 21 h d'incubation.

La quantité d'acide sinapique libérée pour chacune des conditions opératoires est montrée sur la figure 1. Sur cette figure, la référence correspond à la quantité obtenue par hydrolyse alcaline de tourteau de colza, et est indicative de la quantité initiale d'acide sinapique dans les tourteaux de colza.

Comme on peut le voir, de l'acide sinapique est libéré dans le surnageant de culture quelle que soit la préparation enzymatique mise en œuvre. Le meilleur résultat est obtenu pour le bouillon de culture de la souche *d'Aspergillus niger* sur tourteau de colza, après 4 h d'incubation : on obtient 0,47 g d'acide sinapique par 100 g de tourteau de colza. Ceci correspond à 78 % du contenu initial d'acide sinapique dans le tourteau (référence : quantité mesurée à 0,6 g par 100 g de tourteau).

A l'issue de cette étape, l'acide sinapique est séparé du surnageant de culture.

### B.2/ Libération d'acide sinapique de tourteaux de colza par des bouillons de culture d'Asperaillus niger ou des enzymes pures Fae A ou Chlo E

100 mL de surnageant d'une culture de la souche d'*A*. *niger* BRFM 281 réalisée en présence de 15 g/L de RSM4 ou de SFM1 (toutes fractions), selon les conditions décrites au point B.1/ ci-avant, sont récupérés après 4 jours d'incubation.

Ces surnageants contiennent : 30,5 nkat de Fae A, 26,1 nkat de Fae B et 18,2 nkat de Chlo E pour la culture réalisée en présence de RSM4 ; et 13,5 nkat de Fae A, 35,6 nkat de Fae B et 11,4 nkat de Chlo E pour la culture réalisée en présence de SFM1.

Chacun de ces surnageants est incubé à 37 °C et 150tr/min avec 5 g de tourteau de colza RSM4 (toutes fractions).

Alternativement, 100 mL de tampon MOPS 100 mM pH 5,5 contenant soit 30,5 nkat de Fae A pure, soit 18 nkat de Chlo E pure, sont incubés à 37 °C et 150 tr/min avec 5 g de tourteau de colza RSM4 (toutes fractions).

Le témoin est constitué de 100 mL de tampon MOPS 100 mM pH 5,5 dépourvu d'enzymes et incubé à 37 °C, 150 tr/min avec 5 g de tourteau de colza RSM4.

La libération de l'acide sinapique et la disparition de la sinapine sont mesurées par analyse HPLC, selon la méthode indiquée ci-avant, après respectivement 0, 2 et 4 heures d'incubation.

La quantité d'acide sinapique libéré pour chacune des conditions opératoires est montrée sur la figure 2. Comme on peut l'observer, de l'acide sinapique est libéré au bout de 2 heures en présence des préparations enzymatiques issues de la souche d'*A. niger* BRFM 281, ou en présence de Fae A pure. Le meilleur résultat est obtenu pour la Fae A pure après 4 heures d'incubation : on obtient 0,50 g d'acide sinapique pour 100 g de tourteau de colza. Les bouillons de culture d'*A*. *niger* obtenus conformément à l'invention permettent également de libérer une quantité importante d'acide sinapique des tourteaux de colza.

La quantité de sinapine libérée pour chacune des conditions opératoires est montrée sur la figure 3. On en déduit que la libération d'acide sinapique est concomitante à la disparition de sinapine, qui est hydrolysée quasi instantanément après sa libération du tourteau, et ce quelle que soit la préparation enzymatique mise en œuvre, exception faite de l'enzyme Chlo E.

### B.3/ Biotransformation d'acide sinapique en canolol

Cette expérience est réalisée à partir d'acide sinapique commercial.

L'acide sinapique (SA) est ajouté à des cultures de 3 jours de *N. lepideus* BRFM 15 et, ensuite, ajouté quotidiennement pour maintenir une concentration finale de 300 à 400 mg.l⁻¹ dans le milieu de culture.

Le composé principal formé détecté dans le milieu de culture est le canolol, ce qui est confirmé par spectrométrie de masse.

La concentration de chacun des composés aromatiques acide sinapique et canolol présents dans le milieu de culture est évaluée en fonction du temps d'incubation. Les résultats sont montrés sur la figure 4.

La production de canolol a débuté au 4^{ème} jour de culture pour atteindre la concentration maximale de 1,123 g.l⁻¹ au jour 11 avec une productivité de 102 mg.l⁻¹.jour⁻¹ et un rendement molaire de 75 %. Pendant la biotransformation d'acide sinapique en canolol, d'autres composés phénoliques ont été détectés comme formés en quantités mineures : acide syringique (SGA) et syringaldéhyde (SGAL).

### B.4/ Biotransformation en canolol d'acide sinapique naturel issu de l'hydrolyse de tourteau de colza par N. lepideus BRFM 15

Cette expérience est réalisée avec de l'acide sinapique naturel issu de l'hydrolyse de tourteau de colza. Elle comprend deux étapes :
- l'hydrolyse des formes estérifiées de l'acide sinapique présentes dans le tourteau de colza par la Fae A, suivie de la récupération de l'acide sinapique sur une résine sélective et de son élution par un solvant « vert » facilement évaporable à faible température,
- la bioconversion de cet acide sinapique naturel en canolol grâce à une culture de *N. lepideus* BRFM 15.

Dans la première étape, 814 g (matière sèche) de tourteau de colza ont été incubés avec 35057 nkat de FaeA (soit 39 nkat FaeA par gramme de tourteau) dans 7 l de tampon MOPS 100 mM pH 5, à 55 °C pendant 1 à 4h30 (préférentiellement 1h30) avec une agitation de 500 rpm. A l'issue de cette étape, 5,1 g d'acide sinapique sont libérés par hydrolyse du tourteau, soit un rendement d'hydrolyse de 85-90 %.

Le tourteau résiduel est séparé du surnageant d'hydrolyse enzymatique dont le pH est ajusté à 4. Ce dernier est alors incubé avec un adsorbant hydrophobe spécifique de type copolymère de styrène ou de styrène et divinylbenzène (résine Amberlite® XAD2 ou XAD1180) pour récupérer spécifiquement l'acide sinapique, à raison de 10 à 125 g de résine, préférentiellement 30 à 100 g, par litre de surnageant. La durée d'incubation est de 2 à 18 h, préférentiellement 2 à 4 h, à une température de 30 °C, sous agitation orbitale de 160 rpm. L'élution de l'acide sinapique se fait à l'aide d'éthanol, à raison de 1 à 100 ml d'éthanol, préférentiellement 10 à 50 ml, par gramme de résine.

A l'issue de cette étape, 4,13 g d'acide sinapique sont récupérés, soit un rendement de 81 % par rapport à la quantité d'acide sinapique contenue dans le tourteau. L'éthanol est évaporé sous vide à 30 °C de sorte à obtenir une solution éthanolique d'acide sinapique naturel de concentration 25 g.l⁻¹.

Dans la seconde étape, cette solution d'acide sinapique naturel est ajoutée à des cultures de 3 jours de *N. lepideus* BRFM 15 et, ensuite, ajoutée quotidiennement pour maintenir une concentration finale de 300 à 400 mg.l⁻¹ d'acide sinapique dans le milieu de culture. La concentration de chacun des composés aromatiques formés, acide syringique et canolol, présents dans le milieu de culture, est évaluée en fonction du temps d'incubation.

A titre comparatif, la même expérience est réalisée à partir d'acide sinapique commercial, en solution aqueuse ou en solution éthanolique, à la même concentration.

Les résultats sont montrés sur la figure 13.

La production de canolol a débuté au 4^{ème} jour de culture pour atteindre la concentration maximale de 1,277 g.l⁻¹ au jour 13 avec une productivité de 100 mg.l⁻¹.jour⁻¹ et un rendement molaire de 80 %.

On observe en outre que l'apport d'acide sinapique naturel sous forme de solution éthanolique permet de diminuer d'un facteur 1,6 à 2 la production d'acide syringique, dans les premiers jours de culture, par rapport à une biotransformation réalisée avec de l'acide sinapique dissous dans l'eau.

### C/ Biotransformation d'acide férulique en 4-vinylguaiacol

L'acide férulique (FA) est ajouté à des cultures de 3 jours de *N. lepideus* BRFM15 et, ensuite, ajouté quotidiennement pour maintenir une concentration finale de 300 à 400 mg.l⁻¹ dans le milieu de culture.

Le composé principal détecté dans le milieu de culture est le 4-VG (4-vinylguaiacol), ce qui est confirmé par spectrométrie de masse. Plusieurs essais sont réalisés et les concentrations finales en 4-VG varient de 1,1 à 1,85 g.l⁻¹, ce qui est le seuil de solubilité maximale pour le 4-VG en phase aqueuse.

La concentration de chacun des composés aromatiques, acide férulique et 4-VG, présents dans le milieu de culture est évaluée en fonction du temps d'incubation. Les résultats sont montrés sur la figure 5.

Par exemple, 1,433 g.l⁻¹ de 4-VG ont été produits en 16 jours de culture. La production of 4-VG a débuté au 4^{ème} jour de culture et la productivité maximale (95 mg.l⁻¹.jour⁻¹) atteinte au jour 14. Le rendement molaire a été d'environ 100%.

### D/ Biotransformation d'acide caféique en 4-vinvlcatéchol

L'acide caféique est ajouté à des cultures de 3 jours de *N. lepideus* BRFM15 et, ensuite, ajouté quotidiennement pour maintenir une concentration finale de 300 à 400 mg.l⁻¹ dans le milieu de culture.

Le composé principal détecté dans le milieu de culture est le 4-vinylcatéchol, ce qui est confirmé par spectrométrie de masse.

### E/ Biotransformation d'acide férulique en présence de résine adsorbante

### 1^{ère} Expérience

La biotransformation d'acide férulique (FA) en 4-VG est réalisée par un procédé conforme à l'invention mettant en œuvre la résine adsorbante HP20 (copolymère de styrène et de divinylbenzène, commercialisé par Mitsubishi).

La concentration de FA est maintenue quotidiennement à 300 mg.l⁻¹ par supplémentation adéquate pendant 14 jours de culture. L'adsorbant (100 g.l⁻¹) est ajouté directement sous forme de particules libres au jour 4 de culture. Puis, aux jours 7, 10 et 14 de culture, le milieu de culture et la résine sont éliminés des fioles et séparés par filtration. Les métabolites adsorbés sur la résine sont extraits 2 fois par l'éthanol pur, par agitation pendant 2h à 200-300 tr/min à 30 °C.

Les rendements en 4-VG sont évalués en absence et en présence de résine HP20. En l'absence de résine, le rendement molaire de bioconversion de FA en 4-VG est de 71 % (0,7 g.l⁻¹, c'est-à-dire 4,6 mmol.l⁻¹ de 4-VG produit pour 6,5 mmol.l⁻¹ de FA consommé). En présence de résine HP20, le rendement molaire augmente à 82 % avec une augmentation de 2,2 fois de la quantité de 4-VG produit (1,57 g.l⁻¹, c'est-à-dire 10,4 mmol.l⁻¹ de 4-VG). Seuls 13 % du FA total ajouté a été adsorbé sur la résine.

### 2^{ème} expérience

Dans cette expérience, il est mis en œuvre la résine adsorbante XAD2 (copolymère de styrène et de divinylbenzène).

Après 5 jours de culture dans les conditions indiquées dans la 1^{ère} expérience ci-avant (4,6 L de culture), de la résine XAD2 est ajoutée dans le milieu de culture à raison de 100 g/L. Le 4-vinylguaiacol est adsorbé sur la résine au fur et à mesure de sa production dans le milieu de culture. Après 13 jours de culture, la résine est séparée du milieu et le 4-vinylguaiacol est élué avec du méthanol (ou de l'éthanol), de sorte à obtenir 200 mL de solution alcoolique concentrée contenant 8,9 g de 4-vinylguaiacol à une pureté de 99 %. Le rendement molaire est de 70 %.

### F/ Biotransformation d'acide sinapique en présence de résine adsorbante

La biotransformation d'acide sinapique (SA) en canolol est mise en œuvre par un procédé conforme à l'invention mettant en œuvre la résine adsorbante XAD2 (copolymère de styrène et de divinylbenzène).

La concentration d'acide sinapique est maintenue quotidiennement à 300 mg.l⁻¹ par supplémentation adéquate pendant 14 jours de culture.

Après 13 jours de culture, le surnageant (4,2L) est séparé du mycélium, ajusté à pH 7 et ajouté à 420 g de résine XAD2. Après adsorption sur la résine, le canolol est élué avec du méthanol, de sorte à obtenir 200 mL de solution alcoolique concentrée contenant 2,2 g de canolol à une pureté de 70 %. Le rendement molaire est de 70 %.

### G/ Biotransformation d'acides hydroxycinnamiques par différentes souches de Neolentinus lepideus et d'autres champignons

### G.1/ Expérience 1

Cette expérience est réalisée dans les conditions opératoires décrites au point B.3/ ci-dessus, à la différence qu'il est mis en œuvre une concentration en acide hydroxycinnamique de 150 mg/L au lieu de 300 mg/L.

Les souches de *N. lepideus* mises en œuvre sont la souche BRFM 15 et la souche HHB14362-Sp.

Les deux souches *N. lepideus* BRFM 15 et *N. lepideus* HHB14362-Sp sont cultivées en présence soit d'acide sinapique, soit d'acide férulique, soit d'acide p-coumarique comme précurseurs, respectivement, de canolol, de 4-vinylguaiacol et de 4-vinylphénol. Dans cette expérience, chaque précurseur est ajouté à partir du troisième jour de culture et de sorte à en maintenir une concentration de 150 mg/L dans le milieu de culture. Les concentrations de chacun des composés aromatiques présents dans le milieu de culture sont évaluées en fonction des temps d'incubation pour les deux souches testées. Les résultats sont montrés sur les figures 6, 7 et 8 respectivement pour la biotransformation de l'acide sinapique, l'acide férulique et l'acide p-coumarique.

Il apparait de ces figures les constatations suivantes.

La production de canolol a débuté au 4^{ème} jour de culture pour atteindre la concentration maximale de 420 mg/L au jour J11 avec un rendement molaire de 45 % pour la souche BRFM 15, et 319 mg/L au jour J11 avec un rendement molaire de 46 % pour la souche HHB14362-Sp.

La production de 4-vinylguaiacol a débuté au 4^{ème} jour de culture pour atteindre la concentration maximale de 782 mg/L au jour J13 avec un rendement molaire de 49 % pour la souche BRFM 15, et 609 mg/L au jour J14 avec un rendement molaire de 34 % pour la souche HHB14362-Sp.

La production de 4-vinylphénol a débuté au 4^{ème} jour de culture pour atteindre la concentration maximale de 340 mg/L au jour J10 avec un rendement molaire de 35 % pour la souche BRFM 15, et 180 mg/L au jour J10 avec un rendement molaire de 21 % pour la souche HHB14362-Sp.

Ainsi, les deux souches de *N. lepideus* transforment efficacement les trois composés testés.

### G.2/ Expérience 2

Cette expérience est réalisée dans les conditions opératoires décrites au point B.3/ ci-dessus, pour les acides hydroxycinnamiques que sont l'acide férulique et l'acide sinapique.

Les souches de *N. lepideus* mises en œuvre sont la souche BRFM 15 et la souche HHB14362-Sp décrites ci-avant, ainsi que les deux souches suivantes, appartenant au sous-groupe des *Agaricomycotina* des champignons du groupe des *Basidiomycota* :
- souche de l'espèce *Schizophyllum commune*, déposée dans la collection CIRM-BRFM sous le numéro d'accession BRFM 823,
- souche de l'espèce *Stereum hirsutum*, déposée dans la collection CIRM-BRFM sous le numéro d'accession BRFM 889.

Ces espèces ont été annotées sur le site du JGI (Joint Genome Institute) comme possédant des PAD putatives.

Les quatre souches sont cultivées en présence soit d'acide sinapique, soit d'acide férulique, comme précurseurs, respectivement, de canolol et de 4-vinylguaiacol. Dans cette expérience, chaque précurseur est ajouté à partir du troisième jour de culture et de sorte à en maintenir une concentration de 300 mg/L dans le milieu de culture, à l'exception, pour les souches que sont *S. commune* BRFM 823 et *S. hirsutum* BRFM 889, lorsque le précurseur est l'acide sinapique : la concentration en acide sinapique dans le milieu de culture est alors maintenue à 150 mg/L.

Les concentrations de chacun des composés formés dans le milieu de culture (4-vinylguaiacol et canolol) sont évaluées en fonction des temps d'incubation pour les quatre souches testées. Les résultats sont montrés sur les figures 9 et 10 respectivement pour la biotransformation de l'acide férulique et de l'acide sinapique.

Il apparait de ces figures les constatations suivantes.

Les souches de *S. commune* et *S. hirsutum* sont capables de réaliser la biotransformation de l'acide férulique en 4-vinylguaiacol (4-VG), mais pas du tout l'acide sinapique en canolol.

La production de 4-VG démarre au 4^{ème} jour de culture, pour atteindre : 1,45 g/L au jour J14 (productivité 103 mg/L.j), avec un rendement molaire de 87% pour la souche *N. lepideus* BRFM 15 ; 1,03 g/L au jour J15 (productivité 69 mg/L.j), avec un rendement molaire de 71% pour la souche *S. commune* BRFM 823 ; et 69 mg/L au jour J16 (productivité 13 mg/L.j), avec un rendement molaire de 60% pour la souche *S. hirsutum* BRFM 889. Dans tous les cas, les quantités et les rendements obtenus en 4-VG restent inférieurs, pour les souches de *S. commune* et *S. hirsutum,* à ceux obtenus avec les souches de *N. lepideus.*

Ainsi, les enzymes PAD de *S. commune* et *S. hirsutum* sont différentes de celle de *N.* lepideus, et elles présentent une affinité pour l'acide férulique, mais pas du tout pour l'acide sinapique, tout comme les PAD bactériennes.

Par ailleurs, en ce qui concerne les deux souches de *N. lepideus*, on constate que les rendements de production de canolol restent inférieurs, pour la souche *N. lepideus* HHB14362-sp, que pour la souche BRFM 15. Ainsi, la production de canolol démarre au 4^{ème} jour de culture pour atteindre 1,5 g/L au jour J14 (productivité 108 mg/L.j), avec un rendement molaire de 81%, pour la souche *N. lepideus* BRFM 15 ; et 472 mg/L au jour J13 (productivité 33 mg/L.j), avec un rendement molaire de 40%, pour la souche *N. lepideus* HHB14362-sp.

On peut conclure de cette expérience que les deux souches de *N. lepideus* transforment efficacement les deux composés testés, la souche HHB14362-sp étant moins efficace que la souche BRFM 15, alors que les souches S. *commune* BRFM 823 et S. *hirsutum* BRFM 889 ne présentent aucune activité vis-à-vis de la biotransformation de l'acide sinapique, et une activité de biotransformation de l'acide férulique réduite par rapport aux souches de *N. lepideus.*

### H/ Libération d'acide sinapique de tourteaux de colza par l'enzyme FaeA d'Aspergillus niaer- optimisation des conditions

### H.1/ Matériels et méthodes

La quantité totale de sinapine et d'acide sinapique (AS) présents initialement dans le tourteau brut a été déterminée par hydrolyse alcaline. Un gramme de tourteau de colza, préalablement broyé à l'aide d'un broyeur à couteaux IKA®-A11 Basic (Ika-Werke), est extrait trois fois avec 8 ml de méthanol 70% sous agitation à température ambiante pendant 20 min. Les extraits méthanoliques sont regroupés et analysés par HPLC puis mélangés avec 20 ml de NaOH 4M pendant 2 h à 30 °C sous agitation (160 rpm). Le mélange est ensuite acidifié jusqu'à un pH de 1 avec de l'HCl 37% puis analysé par HPLC.

Un lot de FaeA recombinante produite selon Record et al., Appl Microbiol Biotechnol, 2003; 62: 349-355, a été utilisé. 100 ml de tampon MOPS 100 mM pH 5,5 contenant 5 g de tourteau de colza sont incubés à 150 rpm et à des températures comprises entre 30°C et 55°C pendant 1 h pour homogénéiser le mélange, puis des quantités de FaeA comprises entre 30,5 et 196 nkat sont ajoutées.

Le témoin est constitué de 100 ml de tampon MOPS 100 mM pH 5,5, dépourvu d'enzyme, et incubé à 37, 45 ou 55°C et 150 rpm avec 5 g de tourteau de colza.

La libération d'acide sinapique depuis le tourteau est suivie par analyse HPLC, après des temps d'incubation de 1, 2, 3 et 4 h.

### H.2/ Résultats

La composition initiale en sinapine et acide sinapique du tourteau de colza a été déterminée après une extraction méthanolique des composés phénoliques du tourteau suivie d'une hydrolyse alcaline. Les résultats ont montré que le tourteau contient 0,53 % (± 0,01, n=3) de sinapine et 0,74% (± 0,04, n=3) d'acide sinapique.

Les résultats obtenus pour l'hydrolyse enzymatique de la sinapine sont montrés sur la figure 11, pour les différentes températures testées ((a) 37 °C, (b) 45 °C, (c) 55 °C).

Ils démontrent qu'une augmentation de la température de réaction, de même qu'une augmentation de la quantité d'enzyme dans le milieu réactionnel, ont pour effet d'induire une augmentation de la quantité d'acide sinapique libéré du tourteau de colza.

De manière générale, la quantité d'acide sinapique libéré la plus élevée (0,74 %) est obtenue à 55 °C en présence de 196 nkat de FaeA après 3 h d'incubation ; dans ces conditions, le rendement d'hydrolyse est de 100 %. Des taux de libération élevés sont également obtenus par mise en œuvre de 131 nkat d'enzyme.

### I/ Transformation d'acide férulique en 4-vinylguaiacol et d'acide sinapique en canolol par un extrait intracellulaire brut de Neolentinus lepideus BRFM15

### 1.1/ Matériels et méthodes

Une culture de *N. lepideus* BRFM15 est effectuée sur une durée de 11 jours en présence d'acide sinapique comme inducteur de l'enzyme PAD. Au J11, le mycélium est séparé du surnageant par filtration sur filtre 0,22 µm, rincé 3 fois avec 100 ml de tampon sodium phosphate 50 mM pH 6,5. Il est ensuite broyé (broyeur Ultra-Turrax® IKA® S25N-25F, vitesse 13 500 rpm) pendant 3 fois 1 min dans la glace et dans 40 ml de tampon sodium phosphate 100 mM pH 6,8 contenant 650 mM de sorbitol et 1 mM de fluorure de phénylméthylsulfonyle (PMSF), en présence de 30g de sable de Fontainebleau. Le mélange est centrifugé deux fois à 10 000×*g* pendant 30 min à 4 °C pour éliminer les débris cellulaires. Le surnageant de centrifugation constitue l'extrait cellulaire brut (EC) contenant la PAD.

La réaction de biotransformation a lieu à 37 °C pendant 1 à 2 h, dans le milieu réactionnel contenant 50 mM de tampon sodium phosphate pH 6,5 et 2 mM de substrat de bioconversion (acide férulique AF ou acide sinapique AS). Après arrêt de la réaction par ajout d'acide acétique glacial et de méthanol, l'analyse des composés phénoliques (produit de la réaction ; 4-vinylguaiacol ou canolol) est réalisée par HPLC, à 220 nm et 30 °C à l'aide d'un système Agilent1100 (Agilent Technologies), muni d'une colonne C30 phase inverse (YMC® Carotenoid 3 µm, 4.6 × 150 mm, Waters). Le débit d'élution est fixé à 0,8 ml/min. La phase mobile est un mélange d'eau et d'acétonitrile 95:5 (v/v) contenant 0,05% d'acide phosphorique (solvant A) et d'acétonitrile 100% (solvant B). Le gradient est le suivant : 10% B pendant 4 min, amené à 40 % en 9 min, puis à 100% en 1 min jusqu'à la fin de l'analyse (25 min). La quantification se fait par calibration externe. L'activité est exprimée en U/ml d'extrait enzymatique (une Unité U correspond à 1 µmol de produit apparu par heure).

La réaction de biotransformation est réalisée à 30 ou 37 °C, en présence de 1 ou 3 ml d'extrait cellulaire (EC) contenant la PAD.

### 1.2/ Résultats

Les résultats obtenus pour la biotransformation de la sinapine en acide sinapique et de l'acide férulique en 4-vinylguaiacol sont montrés sur la figure 12, pour les différentes températures testées ((a) 30 °C, (b) 37 °C).

Dans toutes les conditions testées, on observe une apparition du produit vinylphénolique (4-vinylguaiacol ou canolol), traduisant l'activité PAD dans l'extrait cellulaire. Au bout de 2 h d'incubation, l'activité PAD est plus élevée à 37 °C qu'à 30 °C. L'activité, en présencede 3 ml d'EC, est 2,5 à 3 fois supérieure à celle mesurée en présence de 1 ml d'EC. A quantité égale d'extrait cellulaire, l'enzyme PAD est particulièrement efficace pour la biotransformation de l'acide férulique.

### J/ Caractéristiques de l'enzyme PAD purifiée du milieu intracellulaire de la souche N. leoideus BRFM15

L'enzyme PAD de la souche sauvage *N. lepideus* BRFM15 est un homodimère de masse moléculaire apparente de 40 ± 2 kDa.

Il ressort des expériences décrites au point I/ ci-avant, ainsi que d'expériences complémentaires, que l'enzyme est active dans une gamme de pH compris entre 5,5 et 7,5, préférentiellement entre 6 et 6,5. Elle est en outre active dans une gamme de températures comprises entre 30 et 55°C, préférentiellement entre 37 et 45°C.

En outre, en présence d'acide férulique et d'acide sinapique, dans un tampon sodium phosphate 50 mM contenant 2 mM de substrat, l'activité maximale est obtenue après 0,5 à 1 h d'incubation. Ainsi, l'activité maximale de la PAD sur l'acide sinapique comme substrat de bioconversion en canolol, à 37 °C et pH 6, est de 7,34± 0,13 U/ml d'enzyme, une unité U correspondant à 1 µmol de canolol apparu par heure. L'activité maximale de la PAD sur l'acide férulique comme substrat de bioconversion en 4-vinylguaiacol, à 45 °C et pH 6,5, est de 46,39 ± 0,46 U/ml d'enzyme, une unité U correspondant à 1 µmol de 4-vinylguaiacol apparu par heure. Ceci correspond à une activité spécifique d'environ 8 U/mg de protéine en présence d'acide sinapique et 51 U/mg de protéine en présence d'acide férulique.

A 37 °C, cette PAD est stable durant 8 h.

A 37 °C et pH 6, il a été déterminé que le K_{M} de la PAD est de 2,65 mM pour l'acide sinapique et 3,23 mM pour l'acide férulique. A 45 °C et pH 6,5, son k_{cat} est de 20 s⁻¹ pour l'acide sinapique et de 135 s⁻¹ pour l'acide férulique.

### K/ Caractéristiques de l'enzyme PAD recombinante purifiée du milieu extracellulaire de la souche recombinante A. niger I-1472-PAD17

L'enzyme PAD de la souche *N. lepideus* HHB14362 a été produite de façon hétérologue chez un champignon théoriquement surproducteur de protéines, *Aspergillus niger.* La PAD native de cette souche est intracellulaire.

### K.1/ Matériels et méthodes

La souche *Aspergillus niger* D15#26 (*pyrG⁻*), protéase-déficiente (décrite dans la publication de Van Hartingsveldt et al., Mol Gen Genetics. 1987, 206: 71-75) a été utilisée pour l'expression hétérologue de la PAD de *N. lepideus* HHB14362. Le gène *pyrG* code l'orotidine-5'-phosphate décarboxylase d'*Aspergillus*, qui est une enzyme impliquée dans la synthèse de l'uridine.

Pour la production hétérologue de la PAD de *N. lepideus* par *A. niger* D15#26, le vecteur d'expression pAN-PAD a été créé à partir du vecteur pAN52-4 (numéro EMBL: Z32699) qui contient également un gène de résistance à l'ampicilline. Dans ce vecteur, la séquence codante *pad* (de séquence nucléotidique SEQ ID NO: 6) est fusionnée à la séquence d'adressage de la glucoamylase d'*A. niger* (24 acides aminés) suivie du site de clivage de la protéase fongique Kex2 (NVISKR).

Deux vecteurs ont été utilisés en co-transformation de la souche *A. niger* D15#26 :
- le vecteur pAN-PAD où l'expression du gène pad est sous le contrôle : *(i)* du promoteur constitutif du gène *gpdA* d'*Aspergillus nidulans*, codant la glycéraldéhyde-3-phosphate déshydrogénase, *(ii)* de la région terminatrice du gène *trpC* d'*A*. *nidulans,* un gène impliqué dans la voie de biosynthèse du tryptophane. Ce type de construction a déjà été utilisé pour la production hétérologue d'autres enzymes fongiques, comme décrit notamment dans la publication de Record et al., Appl Microbiol Biotechnol, 2003, 62: 349-355.
- le vecteur pAB4.1 (décrit dans la publication de Van Hartingsveldt et al., Mol Gen Genetics, 1987, 206: 71-75) qui est un plasmide possédant le gène *pyrG.* La co-transformation de la souche *A. niger* D15#26 (*pyrG⁻*) par ce vecteur annule son auxotrophie pour l'uridine, ce qui permet le criblage des souches fongiques transformées sur milieu dépourvu d'uridine.

### K.2/ Résultats

Cette construction génétique a permis de sécréter la protéine PAD recombinante active dans le milieu extracellulaire. Le clone A. *niger* D15#26-PAD-17, présentant le meilleur niveau de production de PAD (95 U/I), a été sélectionné pour purifier et caractériser la PAD recombinante.

La PAD recombinante est un homodimère de masse moléculaire 40 kDa environ.

Il a été déterminé que son activité est de 2,236 et 0,250 U/ml avec, respectivement, l'acide férulique et l'acide sinapique comme substrats, soit une activité spécifique de 8,28 et 0,93 U/mg respectivement.

L'étude de l'effet du pH et de la température sur l'activité de la PAD recombinante a montré que l'enzyme est active pour des pH compris entre 5,5 et 8, avec un optimum à pH 6-6,5, et pour des températures comprises entre 25 et 55 °C, avec un optimum à 35-40 °C. Il a en outre été déterminé, avec l'acide férulique comme substrat, un K_{M} de 0,33 mM, ce qui reste inférieur à la très grande majorité des K_{M} déterminés pour les PAD de bactéries, traduisant une meilleure affinité de la PAD fongique recombinante vis-à-vis de l'acide férulique.

## Revendications

1. Procédé de préparation d'un composé vinylphénolique à partir d'un acide hydroxycinnamique précurseur, **caractérisé en ce qu'**il comprend une étape de biotransformation dudit acide hydroxycinnamique par mise en présence avec une enzyme à activité décarboxylase d'acide phénolique d'un microorganisme de l'espèce *Neolentinus lepideus.*

2. Procédé selon la revendication 1, selon lequel ladite enzyme présente au moins 90 %, de préférence au moins 95 %, d'identité en amino acides avec la séquence SEQ ID NO: 1.

3. Procédé selon l'une quelconque des revendications 1 à 2, selon lequel l'étape de biotransformation de l'acide hydroxycinnamique est réalisée par culture de cellules dudit microorganisme de l'espèce *Neolentinus lepideus* en présence dudit acide hydroxycinnamique.

4. Procédé selon la revendication 3, mettant en œuvre la souche de *Neolentinus lepideus* déposée sous le numéro ATCC 36557 à l'ATCC.

5. Procédé selon l'une quelconque des revendications 1 à 4, selon lequel ladite étape de biotransformation de l'acide hydroxycinnamique est réalisée en présence dans le milieu réactionnel d'une résine apte à adsorber le composé vinylphénolique formé au fur et à mesure de sa production, notamment un copolymère de styrène et de divinylbenzène.

6. Procédé selon l'une quelconque des revendications 1 à 5, selon lequel l'acide hydroxycinnamique est l'acide sinapique et le composé vinylphénolique est le canolol.

7. Procédé selon l'une quelconque des revendications 1 à 5, selon lequel l'acide hydroxycinnamique est l'acide caféique et le composé vinylphénolique est le 4-vinylcatéchol.

8. Procédé selon l'une quelconque des revendications 1 à 5, selon lequel l'acide hydroxycinnamique est l'acide férulique et le composé vinylphénolique est le 4-vinylguaiacol.

9. Procédé selon l'une quelconque des revendications 1 à 5, selon lequel l'acide hydroxycinnamique est l'acide p-coumarique et le composé vinylphénolique est le 4-vinylphénol.

10. Procédé de production d'un composé vinylphénolique à partir d'un tourteau d'oléagineux, comprenant :
- une étape de libération dudit tourteau d'oléagineux d'un acide hydroxycinnamique précurseur dudit composé vinylphénolique,
- une étape de transformation de l'acide hydroxycinnamique ainsi libéré en ledit composé vinylphénolique par un procédé selon l'une quelconque des revendications 1 à 9.

11. Procédé de production selon la revendication 10, selon lequel l'oléagineux est choisi parmi le colza, le tournesol et le soja.

12. Procédé de production selon l'une quelconque des revendications 10 à 11, selon lequel l'étape de libération de l'acide hydroxycinnamique du tourteau d'oléagineux est réalisée par mise en présence dudit tourteau d'oléagineux avec une enzyme de type cinnamoyl esterase d'un microorganisme de l'espèce *Aspergillus niger.*

13. Procédé de production selon la revendication 12, selon lequel l'étape de libération de l'acide hydroxycinnamique est réalisée par mise en présence dudit tourteau d'oléagineux avec une préparation enzymatique issue du surnageant d'une culture de cellules dudit microorganisme de l'espèce *Aspergillus niger* en présence d'un substrat naturel choisi parmi les tourteaux d'oléagineux et la pulpe de betterave sucrière.

## Patentansprüche

1. Verfahren zur Herstellung einer Vinylphenolverbindung aus einer Vorläufer-Hydroxyzimtsäure, **dadurch gekennzeichnet, dass** es einen Schritt des Biotransformierens der Hydroxyzimtsäure durch Verbinden mit einem Enzym mit Phenolsäuredecarboxylase-Aktivität eines Mikroorganismus der Spezies *Neolentinus lepideus* umfasst.

2. Verfahren nach Anspruch 1, wobei das Enzym weniger als 90 %, vorzugsweise weniger als 95 % Aminosäureidentität mit der Sequenz SEQ ID Nr.: 1 aufweist.

3. Verfahren nach einem der Ansprüche 1 bis 2, wobei der Schritt des Biotransformierens der Hydroxyzimtsäure durch das Kultivieren von Zellen des Mikroorganismus der Spezies *Neolentinus lepideus* in Anwesenheit der Hydroxyzimtsäure durchgeführt wird.

4. Verfahren nach Anspruch 3, das den Stamm von *Neolentinus lepideus,* hinterlegt unter der Nummer ATCC 36557 bei ATCC, einsetzt.

5. Verfahren nach einem der Ansprüche 1 bis 4, wobei der Schritt des Biotransformierens der Hydroxyzimtsäure in Anwesenheit eines Harzes im Reaktionsmedium durchgeführt wird, das ausgelegt ist, die Vinylphenolverbindung zu adsorbieren, die im Laufe ihrer Herstellung gebildet wird, insbesondere ein Copolymer aus Styrol und Divinylbenzen.

6. Verfahren nach einem der Ansprüche 1 bis 5, wobei die Hydroxyzimtsäure Sinapinsäure ist und die Vinylphenolverbindung Canolol ist.

7. Verfahren nach einem der Ansprüche 1 bis 5, wobei die Hydroxyzimtsäure Kaffeesäure ist und die Vinylphenolverbindung 4-Vinylcatechol ist.

8. Verfahren nach einem der Ansprüche 1 bis 5, wobei die Hydroxyzimtsäure Ferulasäure ist und die Vinylphenolverbindung 4-Vinylguaiacol ist.

9. Verfahren nach einem der Ansprüche 1 bis 5, wobei die Hydroxyzimtsäure p-Coumarsäure ist und die Vinylphenolverbindung 4-Vinylphenol ist.

10. Verfahren zur Herstellung einer Vinylphenolverbindung aus einem Ölsaatkuchen, umfassend:
- einen Schritt des Freisetzens von dem Ölsaatkuchen einer Hydroxyzimtsäure, die eine Vorstufe der Vinylphenolverbindung ist,
- einen Schritt des Transformierens der Hydroxyzimtsäure, die so in der Vinylphenolverbindung durch ein Verfahren nach einem der Ansprüche 1 bis 9 freigesetzt wurde.

11. Verfahren zur Herstellung nach Anspruch 10, wobei die Ölsaat ausgewählt ist aus Raps, Sonnenblume und Soja.

12. Verfahren zur Herstellung nach einem der Ansprüche 10 bis 11, wobei der Schritt des Freisetzens der Hydroxyzimtsäure des Ölsaatkuchens durch Verbinden des Ölsaatkuchens mit einem Enzym vom Typ Cinnamoylesterase eines Mikroorganismus der Spezies *Aspergillus niger* durchgeführt wird.

13. Verfahren zur Herstellung nach Anspruch 12, wobei der Schritt des Freisetzens der Hydroxyzimtsäure durch Verbinden des Ölsaatkuchens mit einer enzymatischen Zubereitung durchgeführt wird, die aus dem Überstand einer Kultur von Zellen des Mikroorganismus der Spezies *Aspergillus niger* in Anwesenheit eines natürlichen Substrats durchgeführt wird, ausgewählt aus den Ölsaatkuchen und der Pulpe der Zuckerrübe.

## Claims

1. Method for preparing a vinylphenolic compound from a precursor hydroxycinnamic acid, **characterised in that** it comprises a step of biotransformation of said hydroxycinnamic acid by placing in the presence of an enzyme with an activity of phenolic acid decarboxylase from a microorganism of the species *Neolentinus lepideus.*

2. Method according to claim 1, according to which said enzyme has at least 90%, preferably at least 95%, aminoacid identity with the sequence SEQ ID NO 1.

3. Method according to any one of claims 1 to 2, according to which the step of biotransformation of the hydroxycinnamic acid is carried out by culture of cells of said microorganism of the species *Neolentinus lepideus* in the presence of said hydroxycinnamic acid.

4. Method according to claim 3, implementing the strain of *Neolentinus lepideus* filed under the number ATCC 36557 with the ATCC.

5. Method according to any one of claims 1 to 4, according to which said step of biotransformation of the hydroxycinnamic acid is carried out in the presence in the reaction medium of a resin capable of adsorbing the vinylphenolic compound formed as it is produced, in particular a copolymer of styrene and of divinylbenzene.

6. Method according to any one of claims 1 to 5, according to which the hydroxycinnamic acid is sinapic acid and the vinylphenolic compound is canolol.

7. Method according to any one of claims 1 to 5, according to which the hydroxycinnamic acid is caffeic acid and the vinylphenolic compound is 4-vinylcatechol.

8. Method according to any one of claims 1 to 5, according to which the hydroxycinnamic acid is ferulic acid and the vinylphenolic compound is 4-vinylguaiacol.

9. Method according to any one of claims 1 to 5, according to which the hydroxycinnamic acid is p-coumaric acid and the vinylphenolic compound is 4-vinylphenol.

10. Method for producing a vinylphenolic compound from an oilseed cake, comprising:
- a step of releasing a hydroxycinnamic acid that is a precursor of said vinylphenolic compound from said oilseed cake,
- a step of transforming the hydroxycinnamic acid thus released into said vinylphenolic compound by a method according to any one of claims 1 to 9.

11. Production method according to claim 10, according to which the oilseed is chosen from rapeseed, sunflower and soybean.

12. Production method according to any one of claims 10 to 11, according to which the step of releasing the hydroxycinnamic acid from the oilseed cake is carried out by placing said oilseed cake in the presence of an enzyme of the type cinnamoyl esterase from a microorganism of the species *Aspergillus niger.*

13. Production method according to claim 12, according to which the step of releasing the hydroxycinnamic acid is carried out by placing said oilseed cake in the presence of an enzyme preparation coming from the supernatant of a culture of cells of said microorganism of the species *Aspergillus niger* in the presence of a natural substrate chosen from the oilseed cakes and the pulp of sugar beet.
